Europäisches Patentamt

European Patent Office

Office européen des brevets

(11) Publication number: **0 174 166**
**A1**

# (12) EUROPEAN PATENT APPLICATION

(21) Application number: **85306159.6**

(22) Date of filing: **30.08.85**

(51) Int. Cl.⁴: **C 12 N 15/00**
C 07 H 21/04, C 12 P 19/34
C 12 N 1/20, C 12 N 5/00
//C12R1:41

(30) Priority: **31.08.84 US 646899**

(43) Date of publication of application:
**12.03.86 Bulletin 86/11**

(84) Designated Contracting States:
**AT BE CH DE FR GB IT LI LU NL SE**

(71) Applicant: **Agrigenetics Research Associates Limited**
**3375 Mitchell Lane**
**Boulder Colorado(US)**

(72) Inventor: **Chee, Paula P.**
**5010 Retana Drive**
**Madison Wisconsin 53714(US)**

(72) Inventor: **Kemp, John D.**
**6733 Hammersley Road**
**Madison Wisconsin 53719(US)**

(74) Representative: **Fisher, Adrian John et al,**
**Carpmaels & Ransford 43 Bloomsbury Square**
**London WC1A 2RA(GB)**

(54) **Disarmed T-DNA.**

(57) Disarming mutations of *tmr* and *tms* T–DNA genes are disclosed which are useful in an *Agrobacterium tumefaciens* Ti plasmid-based plant transformation vector. As disarmed T–DNA is compatible with standard plant regeneration protocols, such a vector may be used to introduce a plant-expressible foreign gene into a dicot plant. Methods for use of disarmed T–DNA are provided as are morphologically normal transformed plants. The invention is exemplified by introduction of a *Phaseolus vulgaris* lectin gene into tobacco.

Figure 1

## DISARMED T-DNA

### FIELD

The present invention is in the fields of genetic engineering and plant husbandry, and especially provides a means for transforming a plant to contain a foreign gene by way of a plant-transforming prokaryotic plasmid vector, and further provides plant cells transformed by such a vector.

### BACKGROUND

L. W. Ream et al. (1983) Proc. Natl. Acad. Sci. USA 80:1660-1664, disclose a T-DNA, harbored by strain A3004, having a deletion of a Hpa I fragment between positions 7,257 and 9,442 removing sequences from the structural genes for both tmr and tms. Ream et al. further report that when a strain having the tmr/tms deletion and an insertion in tml is inoculated on stems of Kalanchoë tubiflorae opines were made. Ream et al. did not report regeneration of plants from tmr⁻tms⁻ or tmr⁻tms⁻tml⁻ mutants, nor did they demonstrate that plant cells were transformed to contain tmr⁻tms⁻tml⁻ T-DNA.

T. C. Hall et al., U.S. patent application ser. nos. 485,613*and 485,614,* disclose designs of a tmr⁻tms⁻ disarming mutations having substitutions of a cam gene for T-DNA sequences between the position 5,512 Hind III site and the position 9,062 BamH I site, and between the position 3,390 Hind III site and the position 9,062 BamH I site.

### Shuttle Vectors

Shuttle vectors, developed by G. B. Ruvkun and F. M. Ausubel (1981) Nature 289:85-88, provide a way to insert foreign genetic materials into position of choice in a large plasmid, virus, or genome. There are two main problems encountered when dealing with large plasmids or genomes. Firstly, the large plasmids may have many sites for each restriction enzyme. Unique site-specific cleavage reactions are not reproducible and multi-site cleavage reactions followed by ligation lead to great difficulties due to the scrambling of the many fragments whose order and orientation one does not want changed. Secondly, the transformation efficiency

*See EP-A-0122791 and EP-A-0126546 respectively.

0174166

with large DNA plasmids is very low. Shuttle vectors allow one to overcome these difficulties by facilitating the insertion, often in vitro, of the foreign genetic material into a smaller plasmid, followed by transfer, usually by in vivo techniques, to the larger plasmid.

A shuttle vector consists of a DNA molecule, usually a plasmid, capable of being introduced into the ultimate recipient bacteria having a replicon that can be maintained independently therein. It also includes a copy of the fragment of the recipient genome into which the foreign genetic material is to be inserted and a DNA segment coding for a selectable trait, which is also inserted into the recipient genome fragment. The selectable trait ("marker") is conveniently inserted by transposon mutagenesis in vivo or by in vitro use of restriction enzymes and ligases.

The shuttle vector can be introduced into the ultimate recipient cell, typically a bacterium of the family Rhizobiaceae (which contains the genus Agrobacterium), by a tri-parental mating (Ruvkin and Ausubel, supra), direct transfer of a self-mobilizable vector in a bi-parental mating, direct uptake of exogenous DNA by Agrobacterium cells ("transformation", using the conditions of M. Holsters et al. (1978) Molec. Gen. Genet. 163:181-187), by spheroplast fusion of Agrobacterium with another bacterial cell, by uptake of liposome-encapuslated DNA, or infection with a shuttle vector that is based on a virus that is capable of being packaged in vitro. A tri-parental mating, a technique well known to those skilled in the art of manipulation of large plasmids found in members of the family Rhizobiaceae, involves the mating of a strain containing a mobilizable plasmid, which carries genes for plasmid mobilization and conjugative transfer, with the strain containing the shuttle vector. If the shuttle vector is capable of being mobilized by the plasmid genes, the shuttle vector is transferred to the recipient cell containing the large genome, e.g. the Ti or Ri plasmids of Agrobacterium strains.

After the shuttle vector is introduced into the recipient cell, possible events include a double cross-over with one recombinational event on either side of the marker. This homogenotization event will result in transfer of a DNA segment containing the marker to the recipient genome replacing a homologous segment lacking the insert. To select for cells that have lost the original shuttle vector, the shuttle vector must be

incapable of replicating in the ultimate host cell or be incompatible with an independently selectable plasmid pre-existing in the recipient cell. One common means of arranging this is to conjugally introduce another plasmid which is incompatible with the shuttle vector and which carries a different drug resistance marker. Therefore, when one selects for resistance to both drugs, the only surviving cells are those in which the marker on the shuttle vector has recombined with the recipient genome. If the shuttle vector carries an extra marker, one can then screen for and discard strains that contain plasmids resulting from a single cross-over event between the shuttle vector and the recipient plasmid resulting in cointegrates in which the entire shuttle vector is integrated with the recipient plasmid. If the foreign genetic material is inserted into or adjacent to the marker that is selected for, it will also be integrated into the recipient plasmid as a result of the same double recombination. It might also be carried along when inserted into the homologous fragment at a spot not within or adjacent to the marker, but the greater the distance separating the foreign genetic material from the marker, the more likely will be a recombinational event occurring between the foreign genetic material and marker, preventing transfer of the foreign genetic material.

If the shuttle vector is used to introduce a phenotypically dominant trait (e.g. a novel expressible insecticide structural gene, but not an inactivated oncogenic T-DNA gene) one need not rely on a double homologous recombination. The cells resulting from a single cross-over event resulting in cointegrate plasmids can transfer the desired trait into plant cells (A. Caplan et al. (1983) Science 222:815-821; R. B. Horsch et al. (1984) Science 223:496-498). One may even use a variant shuttle vector having a single uninterrupted sequence of T-DNA. However, as the resulting T-DNA will now contain a tandem duplication, one must be vigilant regarding a possible rare deletion of the shuttle vector by a single homologous recombination event occurring between the two homologous sequences in either the Agrobacterium or plant cells.

Shuttle vectors have proved useful in manipulation of Agrobacterium plasmids: see D. J. Garfinkel et al. (1981) Cell 27:143-153; A. J. M. Matzke and M.-D. Chilton (1981) J. Molec. Appl. Genet. 1:39-49; and J. Leemans et al. (1981) J. Molec. Appl. Genet. 1:149-164, who referred to shuttle vectors by the term "intermediate vectors" or "iV".

A recently disclosed variation of the shuttle vector system for inserting changes into large DNA molecules is the "suicide vector". In this system, as described by R. Simon et al. (1983) Biotechnol. 1:784-791, the shuttle vector replicon cannot be maintained independently within the recipient cell. This property eliminates the need to introduce an incompatible plasmid into the recipient cell in order to exclude the shuttle vector as is commonly done during a triparental mating. All vector sequences which do not integrate into some already present DNA effectively "commit suicide" by not being replicated. As can be done with traditional types of shuttle vectors, one may distinguish between double and single homologous by screening for an antibiotic resistance gene which is not between the two regions of homology. Use of suicide vectors to transfer DNA sequences into a Ti plasmid has also been reported by E. Van Haute et al. (1983) EMBO J. 2:411-417; L. Comai et al. (1982) Plant. Molec. Biol. 1:291-300; L. Comai et al. (1983) Plasmid 10:21-30; P. Zambryski et al. (1983) EMBO J. 2:2143-2150; P. Zahm et al. (1984) Mol. Gen. Genet. 194:188-194; and Caplan et al., supra. C. H. Shaw et al. (1983) Gene 28:315-330, report use of a suicide vector to introduce a foreign DNA into a Ti plasmid without also introducing a selectable marker by means of selection of a single homologous recombinant followed by screening for a double homologous recombinant.

An alternative to the use of homologous recombination for introduction of novel DNA sequences into T-DNA involves bacterial transposons. As described in the section Agrobacterium—Genes on the TIP Plasmids, transposons can "jump" into the T-DNA of a TIP plasmid (e.g. see D. J. Garfinkel et al. (1981) Cell 27:143-153). Should the transposon be modified in vitro by the insertion of the novel sequence, that novel DNA can be transferred into the TIP plasmid's T-DNA by the transposon. The TIP can then transfer the novel DNA/transposon/T-DNA combination to a plant cell when it will be stably integrated.

0174166

## Overview of Agrobacterium

Included within the gram-negative bacterial family Rhizobiaceae (which also includes the genus <u>Rhizobium</u>), in the genus <u>Agrobacterium</u>, are the species <u>A</u>. <u>tumefaciens</u> and <u>A</u>. <u>rhizogenes</u>. These species are respectively the causal agents of crown gall disease and hairy root disease of plants. Crown gall is characterized by the growth of a gall of dedifferentiated tissue. Hairy root is a teratoma characterized by inappropriate induction of roots in infected tissue. In both diseases, the inappropriately growing plant tisssue usually produces one or more amino acid derivatives, known as opines, not normally produced by the plant which are catabolized by the infecting bacteria. Known opines have been classified into three main families whose type members are octopine, nopaline, and agropine. The cells of inappropriately growing tissues can be grown in culture, and, under appropriate conditions, be regenerated into whole plants that retain certain transformed phenotypes.

Virulent strains of <u>Agrobacterium</u> harbor large plasmids known as Ti (tumor-inducing) plasmids (pTi) in <u>A</u>. <u>tumefaciens</u> and Ri (root-inducing) plasmids in <u>A</u>. <u>rhizogenes</u> (pRi). Curing a strain of these plasmids results in a loss of pathogenicity. Ti and Ri plasmids both contain DNA sequences, referred to as T-DNA (transferred-DNA), which in tumors are found to be integrated into the genome of the host plant. The T-DNA encodes several transcripts. Mutational studies have shown that some of these are involved in induction of tumorous growth, though the exact phenotype is host-dependent. Mutants in the genes for <u>tml</u>, <u>tmr</u>, and <u>tms</u>, respectively result in large tumors (in tobacco), a propensity to generate roots, and a tendency for shoot induction. The T-DNA also usually encodes the gene(s) for at least one opine synthase, and the Ti plasmids are often classified by the opine which they caused to be synthesized. Each of the T-DNA genes is under control of a T-DNA promoter. The T-DNA promoters resemble eukaryotic promoters in structure, and they appear to function only in the transformed plant cell. The Ti plasmid also carries genes outside the T-DNA region. These genes are involved in functions which include opine catabolism, oncogenicity, agrocin sensitivity, replication, and autotransfer to bacterial cells. The Ri plasmid is organized in a fashion analogous to the Ti plasmid. The set of genes and DNA sequences responsible for transforming the plant cell are hereinafter collectively referred to as the transformation-inducing principle (TIP). The designation TIP therefore includes, but is not limited to, both Ti and Ri plasmids. The integrated segment of a TIP is termed herein "T-DNA" (transferred DNA), whether derived from a Ti plasmid or an Ri plasmid.

M.-D. Chilton (June 1983) Sci. Amer. 248(6):50-59, has recently provided an introductory article on the use of Ti plasmids as vectors. Recent general reviews of Agrobacterium-caused disease include those by D. J. Merlo (1982), Adv. Plant Pathol. 1:139-178; L. W. Ream and M. P. Gordon (1982), Science 218:854-859; M. W. Bevan and M.-D. Chilton (1982), Ann. Rev. Genet. 16:357-384; G. Kahl and J. Schell (1982) Molecular Biology of Plant Tumors; K. A. Barton and M.-D. Chilton (1983) Meth. Enzymol. 101:527-539; and A. Caplan et al. (1983) Science 222:815-821. A number of more specialized reviews can be found in A. Pühler, ed. (1983) Molecular Genetics of the Bacteria-Plant Interaction, including a treatment by D. Tepfer of A. rhizogenes-mediated transformation.

## Infection of Plant Tissues

Plant cells can be transformed by Agrobacterium in a number of methods known in the art which include but are not limited to co-cultivation of plant cells in culture with Agrobacterium, direct infection of a plant, fusion of plant protoplasts with Agrobacterium spheroplasts, direct transformation by uptake of free T-DNA by plant cell protoplasts, transformation of protoplasts having partly regenerated cell walls with intact bacteria, transformation of protoplasts by liposomes containing T-DNA, use of a virus to carry in the T-DNA, microinjection, and the like. Any method will suffice as long as the gene is stably transmitted through mitosis and meiosis.

The infection of plant tissue by Agrobacterium is a simple technique well known to those skilled in the art (for an example, see D. N. Butcher et al. (1980) in Tissue Culture Methods for Plant Pathologists, eds.: D. S. Ingram and J. P. Helgeson, pp. 203-208). Typically a plant is wounded by any of a number of ways, which include cutting with a razor, puncturing with a needle, or rubbing with abrasive. The wound is then inoculated with a solution containing tumor-inducing bacteria. An alternative to the infection of intact plants is the inoculation of pieces of tissues such as potato tuber disks (D. K. Anand and G. T. Heberlein (1977) Amer. J. Bot. 64:153-158) or inverted segments of tobacco stems (K. A. Barton et al. (1983) Cell 32:1033-1043). After induction, the tumors can be placed in tissue culture on media lacking phytohormones. Hormone independent growth is typical of transformed plant tissue and is in great contrast to the usual conditions of growth of such tissue in culture (A. C. Braun (1956) Cancer Res. 16:53-56).

Agrobacterium is also capable of infecting isolated cells and cells grown in culture (L. Marton et al. (1979) Nature 277:129-131) and isolated tobacco mesophyll protoplasts. In the latter technique, after allowing time for partial regeneration of new cell walls, Agrobacterium cells were added to the culture for a time and then killed by the addition of antibiotics. Other workers (R. B. Horsch and R. T. Fraley (1983) in Advances in Gene Technology: Molecular Genetics of Plants and Animals (Miami Winter Symposium 20), eds.: K. Downey et al., p. 576) have reported transformations by co-cultivation of plant and Agrobacterium cells, leading to a high rate (greater than 10%) of calli displaying hormone-independent growth, with 95% of those calli making opines. M. R. Davey et al. (1980) in Ingram and Helgeson, supra, pp. 209-219, describe the infection of older cells that had been regenerated from protoplasts. The transformation frequency of inoculated callus pieces can be increased by addition of an opine or opine precursors (L. M. Cello and W. L. Olsen, U.S. Patent 4,459,355).

Plant protoplasts can be transformed by the direct uptake of TIP plasmids. M. R. Davey et al. (1980) Plant Sci. Lett. 18:307-313, and M. R. Davey et al. (1980) in Ingram and Helgeson, supra, were able to transform Petunia protoplasts with the Ti plasmid in the presence of poly-L-α-ornithine to a phenotype of opine synthesis and hormone-independent growth in culture. It was later shown (J. Draper et al. (1982) Plant and Cell Physiol. 23:451-458; M. R. Davey et al. (1982) in Plant Tissue Culture 1982, ed: A. Fujiwara, pp. 515-516) that polyethelene glycol-stimulated Ti plasmid uptake and that some T-DNA sequences were integrated into the genome. F. A. Krens et al. (1982) Nature 296:72-74, reported similar results using polyethelene glycol following by a calcium shock, though their data suggests that the integrated T-DNA included flanking non-T-DNA Ti plasmid sequences.

An alternative method to obtain DNA uptake involves the use of liposomes. The preparation of DNA-containing liposomes is well known in the art. Preparations for the introduction of Ti-DNA _via_ liposomes have been reported (T. Nagata _et al._ (1982) in Fujiwara, _supra_, pp. 509-510; T. Nagata (1981) Mol. Gen. Genet. 184:161-165). An analogous system involves the fusion of plant and bacterial cells after removal of their respective cell walls. An example of this technique is the transformation of Vinca or Nicotiana protoplast by Agrobacterium or E. coli spheroplasts (S. Hasezawa _et al._ (1981) Mol. Gen. Genet. 182:206-210; R. Hain _et al._ (1984) Plant Cell Rept. 3:60-64). Plant protoplasts can take up cell wall delimited Agrobacterium cells (S. Hasezawa _et al._ (1982) in Fujiwara, _supra_ pp. 517-518).

T-DNA can be transmitted to tissue regenerated from a fusion of two protoplasts, only one of which had been transformed (G. J. Wullems _et al._ (1980) Theor. Appl. Genet. 56:203-208). As detailed in the section on Regeneration of Plants, T-DNA can pass through meiosis and be transmitted to progeny as a simple Mendelian trait.

The host range of crown gall pathogenesis may be influenced by T-DNA-encoded functions (A. Hoekema _et al._ (1984) J. Bacteriol. 158:383-385). R. L. Ausich, European Patent Application 108,580, reports transfer of T-DNA from A. tumefaciens to green algal cells, and expression therein of octopine synthase and Tn5 kanamycin resistance genes.

Regeneration of Plants

Differentiated plant tissues with normal morphology have been obtained from crown gall tumors. A. C. Braun and H. N. Wood (1976) Proc. Natl. Acad. Sci. USA 73:496-500, grafted tobacco teratomas onto normal plants and were able to obtain morphologically normal shoots that could flower, make opines, and grow independently of phytohormones when placed in culture. L. Otten _et al._ (1981) Molec Gen. Genet. 183:209-213, used Tn7 transposon-generated Ti plasmid mutants in the tms (shoot-inducing) locus to create tumors which proliferated shoots. When these shoots were regenerated into plants, they were found to form self-fertile flowers.

The resultant seeds germinated into plants which contained T-DNA and made opines. The tms phenotype, which can be considered suppression of root formation, can be partly overcome by washing of the rooting area and can be bypassed by grafting onto a normal stock (A. Wöstemeyer et al. (1984) Mol. Gen. Genet. 194:500-507). Similar experiments with a tmr (root-inducing) mutant showed that full-length T-DNA could be transmitted through meiosis to progeny, that in those progeny nopaline genes could be expressed, though at variable levels, and that cotransformed yeast alcohol dehydrogenase I gene was not expressed (K. A. Barton et al. (1983) Cell 32:1033-1043). It now appears that regenerated tissues which lack T-DNA sequences are decended from untransformed cells which "contaminate" the tumor (G. Ooms et al. (1982) Cell 30:589-597) or from cells that have deleted some or all T-DNA (F.-M. Yang et al. (1980) In Vitro 16:87-92; F. Yang et al. (1980) Molec. Gen. Genet. 177:707-714; M. Lemmers et al. (1980) J. Mol. Biol. 144:353-376; F. Yang and R. B. Simpson (1981) Proc. Natl. Acad. Sci. USA 78:4151-4155; H. DeGreve et al. (1982) supra; and J. Memelink et al. (1983) Mol. Gen. Genet. 190:516-522).

Genes involved in opine anabolism were capable of passing through meiosis, though the plants were male sterile, and seemingly unaltered T-DNA can be inherited in a Mendelian fashion (G. J. Wullems et al. (1981) Cell 24:719-724; G. Wullems et al. (1982) in Fujiwara, supra; H. DeGreve et al. (1982) Nature 300:752-755). Genetically, tms and functional opine synthase genes are closely linked in regenerated plants (A. Wöstemeyer et al. (1984) Mol. Gen. Genet. 194:500-507). Functional foreign genes are also inherited in a dominant Mendelian manner (R. B. Horsch et al. (1984) Science 223:496-498), as is Ri T-DNA (D. Tepfer (1984) Cell 37:959-967).

The epigenetic state of the plant cells initially transformed can affect regeneration potential (G. M. S. van Slogteren et al. (1983) Plant Mol. Biol. 2:321-333). Recent work by A. N. Binns (1983) Planta 158:272-279, indicates that tumorogenic genes, in this case tmr, can be "shut off" during regeneration and "turned back on" by placing regenerated tissue in culture.

Roots resulting from transformation from A. rhizogenes have proven relatively easy to regenerate directly into plantlets (M.-D. Chilton et al. (1982) Nature 295:432-434), and are easily cloned. Regenerability from hairy roots appears to be dependent on T-DNA copy-number (C. David et al. (1984) Biotechnol. 2:73-76).

## Genes on the TIP Plasmids

The complete sequence of the T-DNA of an octopine-type plasmid found in ATCC 15955, pTi15955, has been reported and includes fourteen open reading frames (ORFs) flanked by eukaryotic transcriptional control sequences (R. F. Barker et al. (1983) Plant Molec. Biol. 2:335-350). The sequence of the $T_L$ region of pTiAch5, which is closely related to pTi15955, has also been reported (J. Gielen et al. (1984) EMBO J. 3:835-846). T-DNA genes do not contain introns (intervening sequences commonly found in eukaryotic genes which are posttranscriptionally spliced out of the messenger precursor during maturation of the mRNA) and do have sequences that resemble eukaryotic transcriptional start and polyadenylation sites.

A number of genes have been identified within the T-DNA of the TIP plasmids. Several octopine plasmid T-DNA transcripts have been mapped (S. B. Gelvin et al. (1982) Proc. Natl. Acad. Sci. USA 79:76-80; L. Willmitzer et al. (1982) EMBO J. 1:139-146; N. Murai and J. D. Kemp (1982) Nucleic Acids Res. 10:1679-1689; S. J. Karcher et al. (1984) Mol. Gen. Genet. 194:159-165) and some functions have been assigned (J. Leemans et al. (1982) EMBO J. 1:147-152). Some of these regions, specifically those encoding tmr and tms, can also be transcribed in prokaryotic cells (G. Schröder et al. (1983) EMBO J. 2:403-409). The Ti plasmid genes tms, tmr, tml, and ocs have been well defined by transposon mutagenesis (D. J. Garfinkel et al. (1981) Cell 27:143-153). Ti plasmids having single mutations in these genes respectively incite tumorous calli of Nicotiana tabacum which generate shoots, proliferate roots, are larger than normal, and do not synthesize octopine. In other hosts, mutants of these genes can induce different phenotypes (see M. W. Bevan and M.-D. Chilton (1982) Ann. Rev. Genet. 16:357-384). The phenotypes of tms and tmr are correlated with differences in the phytohormone levels present in the tumor. The differences in cytokinin:auxin ratios are similar to those which in culture induce shoot or root formation in untransformed callus tissue (D. E. Akiyoshi et al. (1983) Proc. Natl. Acad. Sci. USA 80:407-411; A. N. Binns (1983) Planta 158:272-279; A. Caplan et al. (1983) Science 222:815-821; R. M. Amasino and C. O. Miller (1982) Plant Physiol. 69:389-392). The tms locus encodes enzymes involved in auxin synthesis (D. Inzé et al. (1984) 194:265-274). T-DNA containing a functional gene for tmr alone, but not functional tml alone or either of the two tms transcripts alone, can promote significant tumor growth. The two tms transcripts can together promote tumor growth. Promotion of shoots and roots is respectively stimulated and inhibited by functional tml (L. W. Ream et al. (1983) Proc. Natl. Acad. Sci. USA 80:1660-1664; D. Inzé et al., supra). Mutations in T-DNA genes do not seem to affect the insertion of T-DNA into the plant genome (Leemans et al. (1982) supra; Ream et al. (1983) supra). T-DNA genes need not be located between border sequences (see TIP Plasmid DNA) to promote hormone independent growth (H. Joos et al. (1983) EMBO J. 2:2151-2160).

Octopine Ti plasmids carry an ocs gene which encodes octopine synthase (lysopine dehydrogenase). All of the signals necessary for expression of the ocs gene are found within 295 bp of the ocs transcriptional start site (C. Koncz et al. (1983) EMBO J. 2:1597-1603). P. Dhaese et al. (1983) EMBO J. 2:419-426, reported the utilization of various polyadenylation sites by "transcript 7" (ORF3 of Barker et al., supra) and ocs. The presence of the enzyme octopine synthase within a tissue can protect that tissue from the toxic effect of various amino acid analogs (G. A. Dahl and J. Tempé (1983) Theor. Appl. Genet. 66:233-239; G. A. Dahl et al., US Pat. application ser. no. 532,280,* which is hereby incorporated by reference).

T$_R$-DNA of octopine-type Ti plasmids carries at least three genes involved in synthesis of the agropine family of opines (J. G. Ellis et al. (1984) Mol. Gen. Genet. 195:466-473).

Nopaline Ti plasmids encode the nopaline synthase gene (nos), which has been sequenced by A. Depicker et al. (1982) J. Mol. Appl. Genet. 1:561-573. All of the signals necessary for expression of the nos gene are found within 261 bp of the nos transcriptional start site (C. Koncz et al., supra). A gene for agrocinopine synthase and genes equivalent to tms and tmr have been identified on a nopaline-type plasmid (H. Joos et al. (1983) Cell 32:1057-1067), and a number of transcripts have been

*See EP-A-0140556.

mapped (L. Willmitzer et al. (1983) Cell 32:1045-1056). J. C. McPhersson et al. (1980) Proc. Natl. Acad. Sci. USA 77:2666-2670, reported the in vitro translation of T-DNA encoded mRNAs from crown gall tissues.

Transcription from hairy root T-DNA has also been detected (L. Willmitzer et al. (1982) Mol. Gen. Genet. 186:16-22). Functionally, the hairy root syndrome appears to be equivalent of a crown gall tumor incited by a Ti plasmid mutated in tmr (F. F. White and E. W. Nester (1980) J. Bacteriol. 144:710-720) as tms⁻ Ti plasmids and Ri plasmids can complement each other when inoculated onto plants, resulting in calli capable of hormone-independent growth (G. M. S. van Slogteren (1983) Ph.D. thesis, Rijksuniversiteit te Leiden, Netherlands).

In eukaryotes, methylation (especially of cytosine residues) of DNA is correlated with transcriptional inactivation; genes that are relatively under methylated are transcribed into mRNA. S. B. Gelvin et al. (1983) Nucleic Acids Res. 11:159-174, has found that the T-DNA in crown gall tumors is always present in at least one unmethylated copy. That the same genome may contain numerous other copies of T-DNA which are methylated suggests that the copies of T-DNA in excess of one may sometimes be biologically inert. (See also G. Ooms et al. (1982) Cell 30:589-597.) Treatment of a tumor line with 5-azacytidine results in demethylation of a T-DNA gene which is paralleled by an increase in transcription (A. G. Hepburn et al. (1983) J. Mol. Appl. Genet. 2:315-329). 5-azacytidine treatment or grafting have been shown to activate silent opine genes (G. M. S. van Slogteren (1983) supra.

The Ti plasmid encodes other genes which are outside of the T-DNA region and are necessary for the infection process. (See M. Holsters et al. (1980) Plasmid 3:212-230 for nopaline plasmids; H. De Greve et al. (1981) Plasmid 6:235-248; D. J. Garfinkel and E. W. Nester (1980) J. Bacteriol 144:732-743; and G. Ooms (1980) J. Bacteriol 144:82-91 for octopine plasmids). Most important are the onc genes, which when mutated result in Ti plasmids incapable of oncogenicity. (These loci are also known as vir, for virulence.) Several onc genes have been accurately mapped and have been found to be located in regions conserved among various Ti plasmids (H. J. Klee et al. (1983) J. Bacteriol. 153:878-883; V. N. Iyer et al. (1982) Mol. Gen. Genet. 188:418-424). The onc genes

function in _trans_, being capable of causing the transformation of plant cells with T-DNA of a different plasmid type and physically located on another plasmid (J. Hille _et al._ (1982) Plasmid _7_:107-118; H. J. Klee _et al._ (1982) J. Bacteriol _150_:327-331; A. J. de Framond _et al._ (1983) Biotechnol. _1_:262-269; J. Hille _et al._ (1984) J. Bacteriol. _158_:754-756; and A. Hoekema _et al._ (1984) J. Bacteriol. _158_:383-385). Nopaline Ti DNA has direct repeats of about 25 base pairs immediately adjacent to the left and right borders of the T-DNA which might be involved in either excision from the Ti plasmid or integration into the host genome (N. S. Yadav _et al._ (1982) Proc. Natl. Acad. Sci. USA _79_:6322-6326), and a homologous sequence has been observed adjacent to an octopine T-DNA border (R. B. Simpson _et al._ (1982) Cell _29_:1005-1014). Opine catabolism is specified by the _occ_ and _noc_ genes, respectively, of octopine- and nopaline-type plasmids. The Ti plasmid also encodes functions necessary for its own reproduction including an origin of replication. Ti plasmid transcripts have been detected in _A. tumefaciens_ cells by S. B. Gelvin _et al._ (1981) Plasmid _6_:17-29, who found that T-DNA regions were weakly transcribed along with non-T-DNA sequences. Ti plasmid-determined characteristics have been reviewed by Merlo, _supra_ (see especially Table II therein), and Ream and Gordon, _supra_.

## TIP Plasmid DNA

Different octopine-type Ti plasmids are nearly 100% homologous to each other when examined by DNA hybridization (T. C. Currier and E. W. Nester (1976) J. Bacteriol. _126_:157-165) or restriction enzyme analysis (D. Sciaky _et al._ (1978) Plasmid _1_:238-253). Nopaline-type Ti plasmids have as little as 67% homology to each other (Currier and Nester, _supra_). A survey revealed that different Ri plasmids are very homologous to each other (P. Costantino _et al._ (1981) Plasmid _5_:170-182) but not to Ti plasmids, except for very weak homologies assignable to opine metabolism functions (K. Lahners _et al._ (1984) Plasmid _11_:130-140). Homologies between octopine- and nopaline-type Ti plasmids, one of which overlapped the T-DNA region, were mapped in detail by G. Engler _et al._ (1981) J. Mol. Biol. _152_:183-208. One of the homologous regions also has homology with a _Sym_ plasmid (involved in symbiotic nitrogen fixation) from a species of Rhizobium, a different genus of the family Rhizobiaceae (R. K. Prakash et al. (1982) Plasmid 7:271-280). Part of the T-DNA sequence is very highly conserved between nopaline and octopine plasmids (M.-D. Chilton et al. (1978) Nature 275:147-149; A. Depicker et al. (1978) Nature 275:150-153). Ri plasmids have been shown to have extensive homology among themselves, and to both octopine (F. F. White and E. W. Nester (1980) J. Bacteriol. 144:710-720) and nopaline (G. Risuleo et al. (1982) Plasmid 7:45-51) Ti plasmids, primarily in regions encoding onc genes. Ri T-DNA contains extensive though weak homologies to T-DNA from both types of Ti plasmid (L. Willmitzer et al. (1982) Mol. Gen. Genet. 186:16-22). Plant DNA from uninfected Nicotiana glauca contains sequences, referred to as cT-DNA (cellular T-DNA), that show homology to a portion of the Ri T-DNA (F. F. White et al. (1983) Nature 301:348-350, L. Spano et al. (1982) Plant Molec. Biol. 1:291-300). G. A. Huffman et al. (1984) J. Bacteriol. 157:269-276, have shown that in the region of cross-hybridization, the Ri plasmid pRiA4b is more closely related to a pTiA6 (octopine-type) than pTiT37 (nopaline-type) and that this Ri plasmid appears to carry sequence homologous to tms but not tmr. Their results also suggested that Ri T-DNA may be discontinuous, analogous to the case with octopine T-DNA (see below). The restriction map of pRiA4b was also disclosed by Huffman et al., supra. Weak homologies have been observed between pRi8196 and several Ti plasmids in regions assignable to opine metabolic functions; no nucleic acid cross-hybridization was observed in T-DNA encoding tumor morphology functions (K. Lahners et al. (1984) Plasmid 11:130-140). T-DNA of pTiBo542, an agropine-type Ti plasmid, may also be discontinuous (E. E. Hood et al. (1984) Biotechnol. 2:702-709).

It has been shown that a portion of the Ti (M.-D. Chilton et al. (1977) Cell 11:263-271) or Ri (M.-D. Chilton (1982) Nature 295:432-434; F. F. White et al. (1982) Proc. Natl. Acad. Sci. USA 79:3193-3197; L. Willmitzer (1982) Mol. Gen. Genet. 186:16-22) plasmid is found in the DNA of tumorous plant cells. The transferred DNA is known as T-DNA. T-DNA is integrated into the host DNA (M. F. Thomashow et al. (1980) Proc. Natl. Acad. Sci. USA 77:6448 6452; N. S. Yadav et al. (1980) Nature 287:458-461) at multiple sites (D. Ursic et al. (1983) Mol. Gen. Genet. 190:494-503; J. Memelink et al. (1983) Mol. Gen. Genet. 190:516-522) in the nucleus (M. P. Nuti et al. (1980) Plant Sci. Lett. 18:1-6; L. Willmitzer et al. (1980) Nature 287:359-361; M.-D. Chilton et al.

(1980) Proc. Natl. Acad. Sci. USA 77:4060 4064). Much non-T-DNA Ti plasmid DNA appears to be transferred into the plant cell prior to T-DNA integration (H. Joos et al. (1983) EMBO J. 2:2151-2160).

M. F. Thomashow et al. (1980) Proc. Natl. Acad. Sci. USA 77:6448-6452 and M. F. Thomashow et al. (1980) Cell 19:729-739, found the T-DNA from octopine-type Ti plasmids to have been integrated in two separate sections, $T_L$-DNA and $T_R$-DNA, left and right T-DNAs, respectively. The copy numbers of $T_R$ and $T_L$ can vary in different tumor lines (D. J. Merlo et al. (1980) Molec. Gen. Genet. 177:637-643). A core of T-DNA is highly homologous to nopaline T-DNA (Chilton et al. (1978) supra, and Depicker et al. (1978) supra), is required for tumor maintenance, is found in $T_L$, is generally present in one copy per cell, and codes for the genes tms, tmr, and tml. On the other hand, $T_R$ can be totally dispensed with (M. De Beuckeleer et al. (1981) Molec. Gen. Genet. 183:283-288, G. Ooms et al. (1982) Cell 30:589-597) and can be found in a high copy number (Merlo et al. (1980) supra). G. Ooms et al. (1982) Plasmid 7:15-29, hypothesized that $T_R$ is involved in T-DNA integration, though they find that when $T_R$ is deleted from the Ti plasmid, A. tumefaciens does retain some virulence. G. Ooms et al. (1982) Cell 30:589-597, showed that though T-DNA is occasionally deleted after integration in the plant genome, it is generally stable and that tumors containing a mixture of cells which differ in T-DNA organization are the result of multiple transformation events. The ocs gene is found in $T_L$ but can be deleted from the plant genome without loss of phenotypes related to tumorous growth. Octopine-type T-DNA borders have been sequenced (R. F. Barker et al. (1983) Plant Mol. Biol. 2:335-350), are 24 base pair imperfect direct repeats of each other, and are homologous with direct repeats found at either end of nopaline T-DNA. Plant DNA flanking integrated left border of $T_L$ has been observed to be composed of repeats of T-DNA sequences which are in either direct or inverted orientations (R. B. Simpson et al. (1982) Cell 29:1005-1014). M. Holsters et al. (1983) Mol. Gen. Genet. 190:35-41, found $T_L$ to be integrated in tanden copies separated by a "linker" of about 400 bp originating from both plant and T-DNA sequences.

In contrast to the situation in octopine-type tumors, nopaline T-DNA is integrated into the host genome in one continuous fragment (M. Lemmers

0174166

et al. (1980) J. Mol. Biol. 144:353-376, P. Zambryski et al. (1980)
Science 209:1385-1391). Direct tandem repeats were observed. T-DNA of
plants regenerated from teratomas had minor modifications in the border
fragments of the inserted DNA (Lemmers et al., supra). Sequence analysis
of the junction between the right and left borders revealed a number of
direct repeats and one inverted repeat. The latter spanned the junction
(Zambryski et al. (1980) supra). The left junction was shown to vary by
at least 70 base pairs while the right junction varies no more than a
single nucleotide (P. Zambryski et al. (1982) J. Mol. Appl. Genet. 1:361-
370). Left and right borders in junctions of tandem arrays were separated
by spacers which could be over 130 bp. The spacers were of unknown origin
and contained some T-DNA sequences. T-DNA was found to be integrated into
both repeated and low copy number host sequences. H. Joos et al. (1983)
Cell 32:1057-1067, have shown that virulence is not eliminated after deletion of one of either of the usual nopaline T-DNA border sequences. The
orientation of the right nopaline border can be reversed without loss of
functionality, and a single border sequence may be capable of transforming
closely-linked sequences (M. De Block et al. (1984) EMBO J. 3:1681-1689).

Simpson et al. (1982) supra, and Zambryski et al. (1980) supra have
suggested that the direct repeats in the border regions are involved in
integration of T-DNA into plant DNA. That T-DNA having borders from two
different Ti plasmids are less specifically integrated than are homologous
borders supports this suggestion (G. Ooms et al. (1982) Plant Molec. Biol.
1:265-276).

A chi ($\chi$) site, which in the bacteriophage $\lambda$ augments general recom-
bination in the surrounding DNA as far as 10 kilobases away, is present in
a nopaline Ti plasmid just outside the left end of the T-DNA (N. S.
Yadav et al. (1982) Proc. Natl. Acad. Sci. USA 79:6322-6326) but not in an
octopine Ti plasmid in an equivalent position (R. B. Simpson et al. (1982)
Cell 29:1005-1014). Barker et al., supra, observed two chi sequences,
both well within $T_L$-DNA. The significance of the chi in Ti plasmids is
not known.

-17-

## Manipulations of the TIP Plasmids

As detailed in the section on Shuttle Vectors, technology has been developed for the introduction of altered DNA sequences into desired locations on a TIP plasmid. Transposons can be easily inserted using this technology (D. J. Garfinkel et al. (1981) Cell 27:143-153). A DNA sequence (e.g. a bacterial transposon) inserted into T-DNA in the Ti plasmid can be transferred and integrated into the recipient plant's genome (J.-P. Hernalsteen et al. (1980) Nature 287:654-656), and can then be recovered in a fully functional and seemingly unchanged form (M. Holsters et al. (1982) Mol. Gen. Genet. 185:283-289). Though insertion of foreign DNA has been done with a number of genes from different sources, to date non-dicot foreign genes have not usually been expressed in plant cells under control of their own promoters. Sources of these genes include rabbit β-globin (C. H. Shaw et al. (1983) Gene 23:315-330), alcohol dehydrogenase (Adh) from yeast (K. A. Barton et al. (1983) Cell 32:1033-1043), AdhI (J. Bennetzen, unpublished) from corn, interferon and globin from mammals, and the mammalian virus SV40 (J. Schell, unpublished). However, when the nopaline synthase gene was inserted into octopine T-DNA and transformed into plant tissue, it was found to be fully functional (C. L. Fink (1982) M.S. thesis, University of Wisconsin-Madison). A gene encoding phaseolin, the storage protein found in seeds of the bean Phaseolus vulgaris L., has been transferred into and expressed in sunflower tumors. Transcription started and stopped at the correct positions, and introns were posttranscriptionally processed properly (N. Murai et al. (1983) Science 222:476-482. An intronless gene for the endosperm protein zein, from the monocot Zea mays, is transcribed though not translated in sunflower callus (M. A. Matzke et al. (1984) EMBO J. 3:1525-1531). Expression of a pea ribulose-1,5-bisphosphate carboxylase small subunit gene is light-regulated in transformed petunia cells; the pea small subunit protein produced is correctly processed and sequestered within chloroplasts (R. Broglie et al. (1984) Science 224:838-843). L. Herrera-Estrella et al. (1984) Nature 310:115-120, report that a 900 bp fragment of DNA from the 5'-upstream region of the same gene is sufficient to confer light-inducible expression in tobacco callus to a bacterial chloramphenicol acetyltransferase structural gene.

0174166

Deletions can be generated in a TIP plasmid by several methods. Shuttle vectors can be used to introduce deletions constructed by standard recombinant DNA techniques. Deletions with one predetermined end can be created by the improper excision of transposons (B. P. Koekman et al. (1979) Plasmid 2:347-357 and G. Ooms et al. (1982) Plasmid 7:15-29). J. Hille and R. Schilperoot (1981) Plasmid 6:151-154, have demonstrated that deletions having both ends at predetermined positions can be generated by use of two transposons. The technique can also be used to construct "recombinant DNA" molecules in vivo. P. Zambryski et al. (1983) EMBO J. 2:2143-2150, report use of a vector, deleted for all nopaline T-DNA genes except nos and transcript a, which on tobacco promotes the formation of very small calli and leads to the regeneration of plants having normal morphology.

The nopaline synthase promoter has been used for insertion of DNA segments coding for drug resistance that can be used to select for transformed plant cells. In plant cells, a bacterial kanamycin resistance gene from Tn5 is not transcribed under control of its own promoter (J. D. Kemp et al. (1983) in Genetic Engineering: Applications to Agriculture, (Beltsville Symp. Agric. Res. 7), ed.: L. D. Owens, pp. 215-228; and C. L. Fink (1982) supra). M. W. Bevan et al. (1983) Nature 304:184-187; R. T. Fraley et al. (1983) Proc. Natl. Acad. Sci. USA 80:4803-4807; and L. Herrera-Estrella et al. (1983) EMBO J. 2:987-995, have inserted the kanamycin resistance gene (neomycin phosphotransferase II), or kan (NPT2), from Tn5 behind (i.e. under control of) the nopaline synthase promoter. The constructions were used to transform plant cells which in culture displayed resistance to kanamycin and its analogs such as G418. Herrera-Estrella et al., supra, reported a similar construction, in which a methotrexate resistance gene (dihydrofolate reductase) from Tn7 was placed behind the nos promoter. Transformed cells were resistant to methotrexate, an antagonistic analog of folic acid. (In work utilizing cauliflower mosaic virus (CaMV) as a vector, a dihydrofolate reductase gene from R67 behind the CaMV 35S promoter similarly conferred methotrexate resistance to infected turnip plants (N. Brisson et al. (1984) Nature 310:511-514.) Furthermore, L. Herrera-Estrella et al. (1983) Nature 303:209-213, have obtained expression in plant cells of enzymatic activity for octopine synthase and chloramphenicol acetyltransferase, an enzyme which in bacteria confers resistance to chloramphenicol, by placing the structural genes for these two enzymes under control of nos promoters. The nos/drug resistance combinations are inherited from transformed plants in a Mendelian manner (R. B. Horsch et al. (1984) Science 223:496-498; M. De Block et al. (1984) EMBO J. 3:1681-1689). G. Helmer et al. (1984) Biotechnol. 2:520-527, have created a fusion gene having the promoter and 5'-end of the structural gene of nos fused to E. coli β-galactosidase (lacZ) sequences. Plant tissues transformed with this construction may be recognized by a characteristic color when grown on the appropriate chromogenic substrate.

Murai et al., supra, reported the fusion of the ocs promoter and the 5'-end of the octopine synthase structural gene to the structural gene for the bean seed protein phaseolin. A fusion protein having the amino terminus of octopine synthase and lacking the amino terminus of phaseolin was produced under control of the T-DNA promoter. The introns, which were contributed by the phaseolin sequences, were posttranscriptionally processed properly.

A. J. de Framond et al. (1983) Biotechnol. 1:262-269, has reported on the construction a "mini-Ti plasmid", a plasmid which could be maintained in A. tumefaciens and lacked almost all non-T-DNA pTi sequences. By itself, this plasmid was not able to transform plant cells. However when placed in an A. tumefaciens strain containing an octopine Ti plasmid, tumors were induced which synthesized both octopine and nopaline. Mini-Ti plasmids can also be transferred into plant cells when complemented with a Ti plasmid deleted for its own T-DNA. These results indicated that the non-T-DNA functions acted in trans with T-DNA, that the missing nopaline Ti plasmid functions were complemented by the octopine Ti plasmid, and that the nopaline "mini-Ti" was functional for the transformation of plant cells. A similar pair of complementing plasmids, each containing either octopine T-DNA or onc genes, has been constructed by A. Hoekema et al. (1983) Nature 303:179-180.

Chilton et al. (18 January 1983) 15th Miami Winter Symp., reported on the construction of a "micro-Ti" plasmid made by resectioning the mini-Ti of de Framond et al., supra, with Sma I to delete essentially all of T-DNA but the nopaline synthase gene and the left and right borders. The micro-Ti was inserted into a modified pRK290 plasmid that was missing its
Sma I site, and was employed in a manner similar to mini-Ti, with comparable results.  G. A. Dahl et al., US pat. application ser. no. 532,280
discloses micro-Ti plasmids carrying ocs genes constructed from the T$_L$
region of the octopine-type plasmid pTi15955.

## SUMMARY OF THE INVENTION

A primary object of this invention is to provide a means for transforming a plant to contain a plant-expressible foreign gene.  In pursuance
of this goal, another object is to provide nonrevertable (e.g. deletions
and substitutions) double mutations of the TIP genes tms and tmr, i.e.
disarming mutations, the mutations being well-defined as to their exact
locations and being compatible with regeneration of whole plants from
cultured tissues.  Other objects are to provide means for simultaneously
incorporating the disarming mutation and a plant-expressible foreign gene
into T-DNA, to provide a means to transform plant cells that are compatible with plant regeneration protocols known to the art, and to provide
several means for selecting for the incorporation into T-DNA of the disarming mutation and/or the plant-expressible foreign gene.  Other objects
will be apparant to those skilled in the art from the present disclosure.

The invention disclosed herein provides a disarming mutation, i.e.
deletion or substitution of sequences that are part of both the tmr and
tms.  Disarming substitutions include replacements of T-DNA by foreign
genes which are plant-expressible and bacterial genes specifying resistances to the antibiotics kanamycin and chloramphenicol.  Shuttle vectors
and Ti plasmids carrying disarming mutations are also provided.  Methods
for construction of disarming mutations are taught as are interactions to
be considered when combining various embodiments of the present invention
or when combining the present invention with other processes or structures
known to the art.  Methods suitable for transformation of plant cells with
disarmed T-DNA and methods suitable for regeneration of plants transformed
by the disarmed T-DNA are also taught.

The experimental work presented herein is believed to be the first
disclosure that plant cells are capable of being regenerated into morphologically normal plants after being transformed with T-DNA disarmed by

0174166

inactivation of <u>tmr</u> and <u>tms</u> and no other genetic loci. It is recognized in the art that during recombinant DNA manipulations, a cryptic promoter might possibly come to be placed in front of a structural gene or fragment thereof, the cryptic promoter having position and orientation relative to the structural gene sequences as to allow transcription of those sequences. It is also recognized that interruptions, deletions, or other changes in protein sequence which are expected to interfere with protein function, do not always inactivate the modified protein. Without the results disclosed herein, it is unobvious that morphologically normal plants would indeed be obtained from plant cells transformed by disarmed T-DNA. As disarmed-T-DNA-transformed callus grows more slowly than wild-type-transformed callus, it was also unobvious that the transformation frequency resulting from use of the vectors and methods of the present invention would be as high as was found with undisarmed vectors. High transformation rates permit the recovery of transformed tissue by screening, i.e. testing individual cloned tissue pieces or regenerated plants for a transformed phenotype. Lower transformation rates would necessitate selection of transformed cells, i.e. a mixture of transformed and untransformed cells are subjected to conditions whereby untransformed cells are killed and transformed cells survive without high rates. Plant-expressible selectable markers would have to be present in the T-DNA transformation vectors and transformed cells would have to be subjected to selection steps, thereby increasing the complexity of tissue handling protocols prior to plant regeneration. It is known in the art that increased time and nonphysiological conditions in tissue culture can adversely affect the phenotype of regenerated plants.

The present invention is useful for producing genetically modified plant cells, plant tissues, and whole plants, especially when the transforming disarmed T-DNA also carries a useful structural gene/plant-expressible promoter combination. In particular, the present invention provides a means to insert foreign DNA into a plant genome in a manner consistent with the regeneration of morphologically normal plants. Use of a deletion or substitution to construct a disarming mutation is advantageous as these classes of mutations cannot revert. Other useful aspects of the present invention will be apparent to those of ordinary skill in the art from the present disclosure.

## BRIEF DESCRIPTION OF THE DRAWINGS

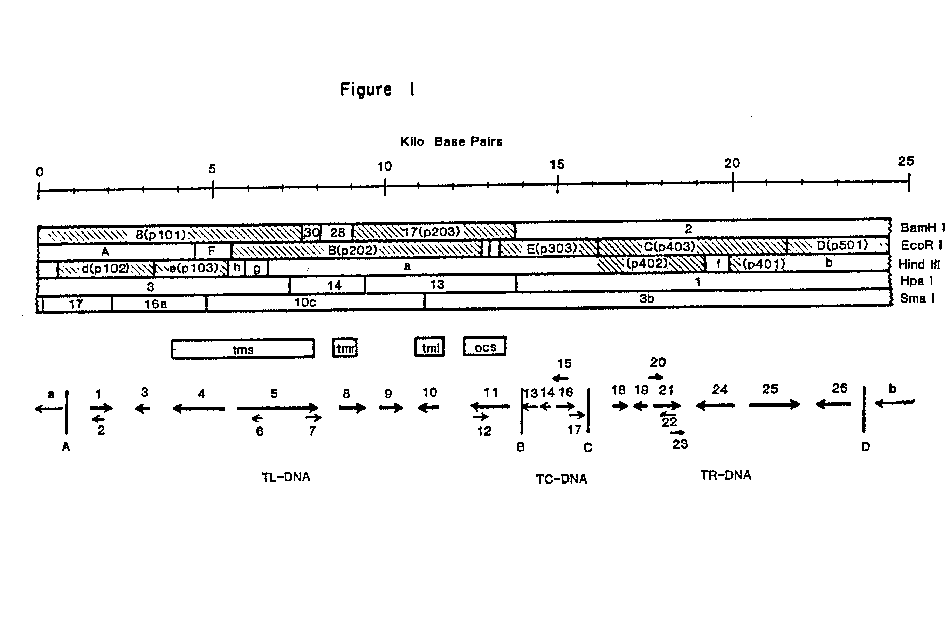

Figure 1, taken from R. F. Barker et al. (1983) Plant Mol. Biol. 2:335-350, is a map of a typical octopine-type Ti plasmid, pTi15955. This map and the sequence presented by Barker et al. is used herein to identify orientations, locations, and regions of T-DNA. On the top is a distance scale measured in units of kilobase pairs (kbp). Below the scale is a map of restriction sites, the lettering therein identifying restriction fragment designations (e.g. BamH I fragment 17 and Hind III fragment e) and plasmids which carry particular fragments (e.g. p103 and p203). Below the restriction map is indicated the locations of genetically defined loci (boxes labeled tms, tmr, tml, and ocs) and open reading frames (numbered and lettered arrows). A, B, C, and D represent T-DNA border sequences.

Figure 2 diagrams assembly of p203-Kan.

Figure 3 diagrams assembly of p203-Kan-103.

Figure 4 diagrams assembly of pRK-203-Kan-103.

Figure 5 diagrams assembly of pRK-203-Kan-103-Lec.

Figure 6 diagrams, schematically and not necessarily to scale, assembly of pRK-203-Cam-103.

## DETAILED DESCRIPTION OF THE INVENTION

The following definitions are provided, in order to remove ambiguity as to the intent or scope of their usage in the specifications and claims.

T-DNA: Refers herein to a segment of DNA derived from the transformation-inducing principle (TIP) which becomes integrated into a plant genome. As used herein, the term includes DNA originally derived from any tumor-inducing strain of Agrobacterium tumefaciens. In addition, as used herein the term T-DNA includes alterations, modifications, mutations, insertions, and deletions, either naturally occurring or introduced by laboratory procedures, a structural requirement being that sufficient of the right and left ends (i.e. borders) of naturally occurring T-DNAs be present to ensure the expected function of stable integration which is characteristic of all T-DNA. Positions within T-DNA are defined as in unmodified T-DNA of pTi15955, as published by R. F. Barker et al. (1983) Plant Mol. Biol. 2:335-350. Positions of unmodified homologous T-DNAs are positions homologous to those defined in pTi15955. Directions are defined so that rightward is in the direction of higher numbered positions. Though T-DNAs other than that of pTi15955 might not have particular restriction sites at exactly the same sites as pTi15955, there may be sites specific to other restriction enzymes reasonably close, i.e. within about one hundred base pairs, to the sites used to construct the disarming mutations disclosed herein; DNA constructions using such sites are within the scope of the present invention as are pTi15955-derived DNA constructions using restriction sites similarly reasonably close to the position 5,512 Hind III site and position 9.062 BamH I site.

T-DNA sequence: Refers herein to any DNA sequence derived from T-DNA as defined above. A T-DNA sequence need not be bound by T-DNA borders.

Disarming mutation: Refers herein to a mutation which causes loss of function of both tmr and tms. Inactivation of either of the two tmr-encoded enzymes is sufficient to cause loss of function of tmr. A disarming mutation may have more than one component, e.g. the term includes insertion into T-DNA of two transposons, one each into tmr and tms. A disarming mutation can exist in a functional T-DNA or in a T-DNA sequence lacking T-DNA borders.

Disarmed T-DNA: Refers herein to a T-DNA lacking functional tmr and tms genes derived from a T-DNA that had functional tmr and tms genes. In most cases, a T-DNA carrying a disarming mutation will be a disarmed T-DNA. Exceptions can include situations where tmr and tms have been duplicated. For example, when a shuttle vector carrying a disarming mutation forms a cointegrate with a T-DNA by single homologous recombination, the tmr-tms region can become duplicated. Although a disarming mutation as defined above will be present, the T-DNA will still carry functional copies of tmr and tms.

Plant tissue: Includes differentiated and undifferentiated tissues of plants including, but not limited to, roots, shoots, pollen, seeds, and tumor tissue, such as crown galls, and various forms of aggregations of plant cells in culture, such as embryos and calluses. The plant tissue may be in planta or in organ, tissue, or cell culture.

Plant cell: As used herein includes plant cells in planta, in plant tissues as defined above, and plant cells and protoplasts in culture.

Plant-expressible foreign gene: Refers herein to a gene that, when in a plant genome, is expressed in at least one tissue during at least one developmental stage but is not found in naturally-occurring T-DNAs.

Plant-detectable marker: Includes genes that when in a plant genome are expressed in cells in culture or in at least one tissue during at least one developmental stage and that facilitates identification of cells, tissues, or plants that have been transformed by a T-DNA carrying the detectable marker. Detectable markers include both selectable markers (e.g. ocs; bacterial antibiotic resistance genes placed behind a plant, plant virus, or T-DNA (i.e. plant-expressible promoter); etc.) and screenable markers (e.g. opine synthases or a plant-expressible promoter/structural gene combination expressing an enzyme that, when provided an appropriate substrate, causes a color change; etc.).

Production of a genetically modified plant transformed by disarmed T-DNA combines the specific teachings of the present disclosure with a variety of techniques and expedients known in the art. The fundamental aspects of the present invention include the construction of disarmed T-DNA, combination of disarmed T-DNA with a plant expressible foreign gene, transformation of a plant cell by the combination, and regeneration of a plant descended from the transformed cell. In most instances, alternative expedients exist for each stage of the overall process. The choice of expedients depends on variables and choices, including the following: T-DNA and T-DNA sequence sources; whether tmr and tms is inactivated by insertion, deletion, or substitution; which plant-expressible foreign gene is to be inserted into the disarmed T-DNA; where that foreign gene is to be inserted; whether a plant-detectable marker is included within the disarmed T-DNA, and if so, which marker and where positioned; how the T-DNA/disarming mutation/foreign gene/detectable marker combination is to be assembled; plant species and variety to be transformed; inoculation method; protocol for culturing transformed tissues; means for detecting which cells are transformed; method for regenerating the transformed tissue to form a morphologically normal plant; and the like, all of which present alternative process steps which those of ordinary skill are able to select and use to achieve a desired result.

As previously discussed and exemplified herein, a tmr⁻ tms⁻ disarming mutation is most advantageously constructed by deleting sequences from those two genes. Two situations that can result from deletions, also previously discussed, are that residual gene product activity may remain even though tmr or tms sequences are missing and that cryptic promoters may be inadvertantly juxtaposed with a structural gene. Either situation might lead to a tmr⁺ or tms⁺ phenotype or otherwise interfer with regeneration of transformed plants. The results disclosed herein demonstrate a deletion of T-DNA sequences between the position 5,512 Hind III site and the position 9,062 BamH I site disarms T-DNA in a manner consistant with the regeneration of transformed plants. In other words, a deletion of T-DNA sequences from a position reasonably close, i.e. within about one hundred base pairs, to the Hind III site towards tmr has a tms⁻ phenotype, and a deletion of T-DNA sequences from a position reasonably close to the BamH I site towards tms has a tmr⁻ phenotype. Deletion of non-pTi15955 T-DNA sequences homologous to the above-specified pTi15955 sequences are within the scope of the present invention. The combination of the tmr⁻ and tms⁻ phenotypes by deletion or substitution of T-DNA sequences between the Hind III and BamH I sites in a transformed plant cell is demonstrated herein to be compatible with regeneration of a whole plant from the transformed cell. With time, disarming mutations bound by other restriction sites may be found to be compatible with regeneration of normal plants; these embodiments are within the scope of the present invention.

In the preferred embodiments, the Hind III and BamH I sites may either be directly ligated to each other, thereby forming a deletion, as exemplified and discussed in Example 7, or may be linked by a DNA fragment having at least one gene, thereby forming a substitution. The substituting DNA may include a bacterial drug resistance gene useful for incorporation by means of marker exchange of the disarming mutation into T-DNA and/or a plant-expressible foreign gene. It is well understood in the art of genetics that a substitution may be considered a deletion of a removed DNA sequence followed by an insertion of other DNA into the resulting 'gap'; in the present invention a substitution is considered to be within the scope of an equivalent deletion, equivalent mutations being ones that remove the same or homologous DNA sequences from an unmodified DNA. Substitutions of a $\underline{kan}$ gene/lectin gene combination and of a $\underline{cam}$ gene are exemplified herein (Examples 4 and 6, respectively).

Alternatively, a plant-expressible foreign gene may be incorporated into T-DNA independently from incorporation of the disarming mutation. As discussed in Example 4, independent incorporation of a foreign gene may be used to inactivate a T-DNA gene in addition to disarming mutation-inactivated $\underline{tmr}$ and $\underline{tms}$. The orientation of the inserted foreign gene, with respect to the direction of transcription and translation of endogenous T-DNA genes is not critical, either of the two possible orientations being functional. Differences in rates of expression may be observed when a given gene is inserted at different locations within T-DNA, possibly because of such factors as chromatin structure. Differences in rates of expression also may vary depending on whether the plant-expressible foreign gene is inserted into disarmed T-DNA in $T_L$ or in $T_R$, as the gene copy number may vary. A single copy of $T_L$ is generally transferred into a plant genome, while multiple copies of $T_R$ can be found in transformed plant tissues. Location of the foreign gene within T-DNA is not critical as long as the transfer function of sequences immediately surrounding the T-DNA borders are not disrupted, since these regions appear from prior art studies to be essential for insertion of the modified T-DNA into the plant genome, and as long as the foreign gene is not placed in $T_C$ (central T-DNA). (Prior art studies have not demonstrated whether $T_C$ is integrated into plant genes; further studies may indicate that $T_C$ is an acceptable alternative to $T_L$ and $T_R$.)

The present invention is useful for facilitating the genetic modification of plant cells, plant tissues, and whole plants by inserting useful foreign structural genes from other species, organisms, varieties, or strains, the structural genes being under control (i.e. behind) plant-expressible promoters. Many useful plant-expressible promoters are known to the art and the discovery of more such promoters is expected in the art. A plant-expressible promoter/structural gene combination constitutes a plant-expressible foreign gene if the combination is incorporated into T-DNA. Considerations concerning combination of promoters and structural genes are understood in the art. Useful structural genes include, but are not limited to, genes conveying phenotypes such as follows: improved tolerance to extremes of heat or cold; improved tolerance to drought or osmotic stress; improved resistance or tolerance to insect (e.g. insecticidal toxins), arachnid, nematode, or epiphyte pests and fungal, bacterial, or viral diseases; the production of enzymes or secondary metabolites not normally found in said tissues or plants; improved nutritional (e.g. storage proteins, lectins, etc.), flavor (e.g. sweet proteins), or processing properties when used for fiber or human or animal food; changed morphological traits or developmental patterns (e.g. leaf hairs which protect the plant from insects, coloring which is aesthetically pleasing, changed plant growth habits, dwarf plants, reduced time needed for the plants to reach maturity, expression of a gene in a tissue or at a time that gene is not usually expressed, and the like); male sterility; improved photosynthetic efficiency (including lowered photo-respiration); improved nitrogen fixation; improved uptake of nutrients; improved tolerance to herbicides; increased crop yield; improved competition with other plants; and improved germplasm identification by the presence of one or more characteristic nucleic acid sequences, proteins, or gene products, or phenotypes however identified (to distinguish a genetically modified plant or the present invention from plants which are not so modified, to facilitate transfer of a linked artificially introduced phenotype by other (e.g. sexual) means to other genotypes or to facilitate identification of plants protected by patents or by plant variety protection certificates); resistance in cell or tissue culture to selective agents (i.e. selectable markers); and the like. The invention is exemplified by the introduction into and expression in plant cells of a Phaseolus vulgaris lectin gene behind its own promoter. The introduction and expression of a novel lectin may be used to change the nutritional or symbiotic properties or a plant tissue. Other uses of the invention, exploiting the properties of other structural genes introduced into various plant species and placed behind various plant-expressible promoters, will be readily apparent to those skilled in the art.

Once designed, a disarming mutation and any disarming mutation/ foreign gene combination is constructed by the methods well known to those of ordinary skill in the art of recombinant DNA. A disarming mutation and/or foreign gene may be incorporated into T-DNA, thereby forming disarmed T-DNA, by means of a shuttle vector, as is well understood and as
has been described in the Background and exemplified in Example 6. The
disarming mutation may be incorporated into any T-DNA with which it has
sufficient homology to recombine with, possibly including T-DNAs which are
not from octopine-type Ti plasmids.

After a _Agrobacterium_ cell harboring a plant-expressible foreign
gene/disarmed T-DNA combination is produced, the cell is used to transform
a plant cell. Methods useful for plant cell transformation are reviewed
in the Background section on Infection of Plant Tissues. In the preferred
embodiments, stem segments are inverted relative to their normal orientation and placed on a solidified medium, as described by K. A. Barton
_et_ _al._ (1983) Cell _32_:1033-1043. The callus resulting after culture of
the inverted stem segments is then preferably removed from the segment and
placed on a medium suitable for callus culture supplemented with a bac-
teriocidal antibiotic capable of killing the inoculating _A._ _tumefaciens_
strain. Other alternatives for producing an _Agrobacterium_-free callus
culture include supplementing the stem culture medium with antibiotic.

As originally produced, a callus is a mosaic of transformed and
untransformed cells. A plant regenerated from such a callus will likewise
be a mosaic. Seed produced from such a plant will be a mixture of transformed and untransformed seed. To increase the genetic uniformity of a
regenerated plant, one may single-cell clone transformed callus. Single-
cell cloning may be accomplished by any means known to the art. Typically, a callus piece known to contain transformed cells is placed in
liquid culture. The resultant suspension culture is sieved to isolate
single cells and very small cell clumps. The resultant filtrate is plated
onto solid medium at a density consistant with growth of the cells or
clumps into callus. As is understood in the art, plating onto a solid
medium containing feeder cells is sometimes advantageous.

Transformed plant tissues may be identified after regeneration from
cloned callus. However, in the preferred embodiments, transformed calli
are identified before plant regeneration. As exemplified herein, one may
screen individual callus pieces of a transformed phenotype. Screening of
transformed tissues for tissues expression the foriegn structural gene may be done by immunological means, including micro-ELISAs (enzyme-linked immuno-sorbant assays), a technique well known to those skilled in the art of immunochemistry, and "Western" blots of antigens after electrophoresis through SDS-polyacrylamide gels (e.g as described by R. P. Legocki and D. P. S. Verma (1981) Analyt. Biochem. 111:385-392). Southern, Northern (e.g., P. S. Thomas (1980) Proc. Natl. Acad. Sci. USA 77:5201-5205) and dot blots, all methods well known to those skilled in the art of molecular biology, may be used to detect incorporated DNA and expressed RNA. Assays for opine production (e.g. octopine and mannopine are characteristic of $T_L$ and $T_R$ from pTi15955, respectively) are also indicative of the presence of T-DNA. Additionally, the phenotype of an expressed foreign gene can be used to identify transformed tissue (e.g. insecticidal properties of an insecticidal toxin). Selection of transformed cells may be done with the drugs and selectable markers described in the Background (Manipulation of the TIP Plasmids). Selection is most readily accomplished by providing a selectable marker incorporated into the T-DNA disarmed foreign gene combination. Examples of selectable markers include either dihydrofolate reductase or neomycin phosphotransferase expressed under control of a nopaline synthase promoter. These markers are selected by growth in medium containing methotrexate or kanamycin, respectively, or their analogs. In addition, the T-DNA can provide an endogenous marker such as a gene controlling resistance to toxic compounds such as amino acid analogs. Such resistance is provided by an opine synthase gene, ocs, to about 10 µg/ml S-(2-aminoethyl)-L-cysteine (AEC) (G. A. Dahl and J. Tempe (1983) Theor. Appl. Genet. 66:233-239, G. A. Dahl et al., U.S. Patent application ser. no. 532,280). The exact drug, drug concentration, plant tissue, plant species, and cultivar must be carefully matched and chosen for ability to regenerate and for efficient selection. Single-cell clones may be selected either by plating a suspension culture directly onto a selective medium or by transferring pieces of cloned callus to a selective medium.

An object of the present invention is to provide a means to transform a plant cell that is compatible with plant regeneration protocols known to the art. After isolation of a transformed callus, plants are regenerated using known methods developed for regeneration of untransformed callus of that species, as exemplified herein with tobacco. However, it is recognized that for some plant species, untransformed tissue regeneration conditions may have to be modified for regeneration of disarmed T-DNA transformed callus. Example 9 demonstrates that disarmed T-DNA transformed tobacco callus may be regenerated by well known methods: shoots are induced from callus grown on an auxin, 1-naphthalene acetic acid (NAA), and a cytokinin (kinetin) by removal of the auxin from the culture medium; subsequently the resultant shoots are induced to root by being cultured on a medium lacking both auxin and cytokinin.

The genotype of the plant tissue transformed is often chosen for the ease with which its cells can be grown and regenerated in in vitro culture and for susceptibility to the selective agent to be used. Should a cultivar of agronomic interest be unsuitable for these manipulations, a more amenable variety is first transformed. After regeneration, the newly introduced disarmed T-DNA/foreign gene combination is readily transferred to the desired agronomic cultivar by techniques well known to those skilled in the arts of plant breeding and plant genetics. Sexual crosses of transformed plants with the agronomic cultivars yielded an initial hybrid. These hybrids can then be back-crossed with plants of the desired genetic background. Progeny are continuously screened and selected for the continued presence of integrated T-DNA or for the new phenotype resulting from expression of the inserted plant-expressible foreign gene. In this manner, after a number of rounds of back-crossing and selection, plants can be produced having a genotype essentially identical to the agronomically desired parents with the addition of the inserted disarmed T-DNA/foreign gene combination.

## EXAMPLES

The following Examples utilize many techniques well known and accessible to those skilled in the arts of molecular biology and manipulation of TIPs and Agrobacterium; such methods are fully described in one or more of the cited references if not described in detail herein. Enzymes are obtained from commerical sources and are used according to the vendor's recommendations or other variations known to the art. Reagents, buffers, and culture conditions are also known to those in the art. Reference works containing such standard techniques include the following: R. Wu, ed. (1979) Meth. Enzymol. <u>68</u>; R. Wu <u>et al</u>., eds. (1983) Meth. Enzymol. <u>100</u> and <u>101</u>; L. Grossman and K. Moldave, eds. (1980) Meth. Enzymol. <u>65</u>; J. H. Miller (1972) <u>Experiments in Molecular Genetics</u>; R. Davis <u>et al</u>. (1980) <u>Advanced Bacterial Genetics</u>; R. F. Schleif and P. C. Wensink (1982) <u>Practical Methods in Molecular Biology</u>; and T. Maniatis <u>et al</u>. (1982) <u>Molecular Cloning</u>. Additionally, R. F. Lathe <u>et al</u>. (1983) Genet. Engin. <u>4</u>:1-56, made useful comments on DNA manipulations.

Textual use of the name of a restriction endonuclease in isolation, e.g. "<u>BamH</u> I", refers to use of that enzyme in an enzymatic digestion, except in a diagram where it can refer to the site of a sequence susceptable to action of that enzyme, e.g. a restriction site. In the text, restriction sites are indicated by the additional use of the word "site", e.g. "<u>BamH</u> I site". The additional use of the word "fragment", e.g. "<u>BamH</u> I fragment", indicates a linear double-stranded DNA molecule having ends generated by action of the named enzyme (e.g. a restriction fragment). A phrase such as "<u>Bcl</u> I/<u>BamH</u> I fragment" indicates that the restriction fragment was generated by the action of two different enzymes, here <u>Bcl</u> I and <u>BamH</u> I, the two ends resulting from the action of different enzymes. Note that the ends will have the characteristics of being "sticky" (i.e. having a single-stranded protrusion capable of base-pairing with a complementary single-stranded oligonucleotide) or "blunt" and that the specificity of a sticky-end will be determined by the specificity of the enzyme which produces it.

Plasmids, and only plasmids, are prefaced with a "p", e.g., pTi15955 or pUC19, and strain designation followed parenthetically by a plasmid name indicates that a plasmid is harbored within that strain, e.g., <u>A</u>. <u>tumefaciens</u> (pTi15955) or <u>E</u>. <u>coli</u> K802 (pUC19). The following strains are on deposit:

| | |
|---|---|
| <u>E</u>. <u>coli</u> C600 (pRK-203-Kan-103-Lec) | NRRL B-15821 |
| <u>A</u>. <u>tumefaciens</u> (pTi15955) | ATCC 15955 |
| <u>E</u>. <u>coli</u> C600 (pKS4) | NRRL B-15394 |
| <u>E</u>. <u>coli</u> HB101 (pPVL134) | ATCC 39181; |

NRRL strains being with the Northern Regional Research Center, 1815 N. University St., Peoria, Illinois 61604 USA, and ATCC strains being with the American Type Culture Colection, 12301 Parklawn Dr., Maryland 20852 USA. Other plasmids and strains are widely available and accessible to those in the art.

Example 1  The right end of the disarming deletion

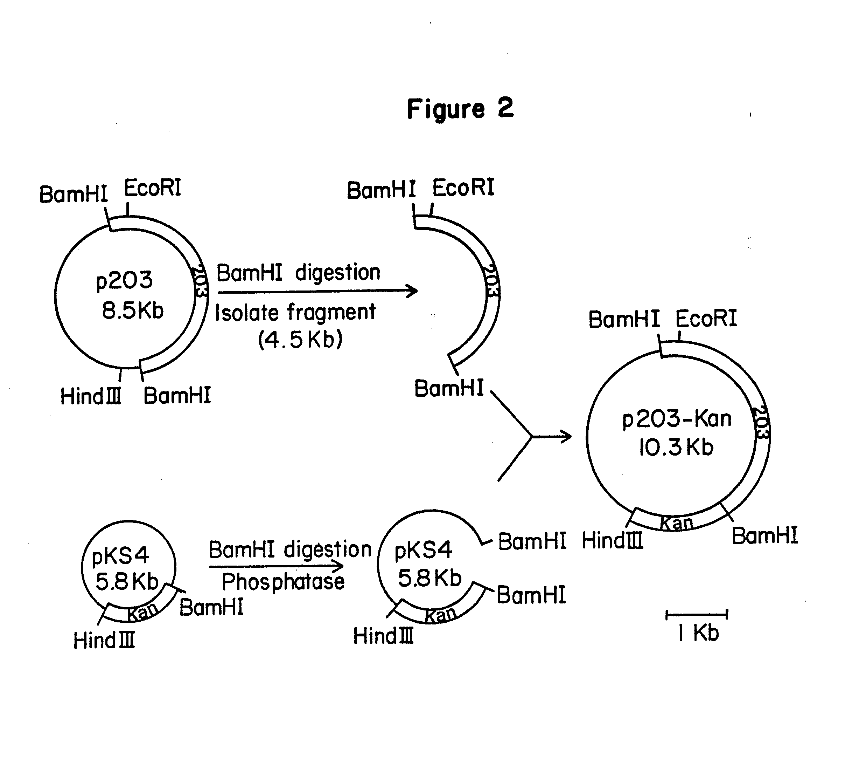

p203 is a plasmid having BamH I fragment 17 of pTi15955 (4.5 kbp) inserted into the BamH I site of pBR322.  p203 was digested with BamH I and the 4.5 kbp T-DNA fragment was isolated by elution from an agarose gel after electrophoretic separation.  After the 4.5 kbp fragment was mixed with and ligated to BamH I-linearized, CIAP-digested pKS4 (which may be isolated from E. coli C600 (pKS4), NRRL B-15394), the reaction mixture was transformed into E. coli HB101.  (CIAP (calf intestinal alkaline phosphatase) dephosphorylates pKS4's BamH I sticky-ends, thereby preventing religation of pKS4 molecules not having T-DNA inserts, a method well known in the art.)  pKS4 is a pBR322 derivative carrying a kanamycin resistance gene (kan, encoding the enzyme neomycin phosphotransferase II) derived from the transposon Tn5.  DNAs isolated from transformants resistant to kanamycin and ampicillin were characterized by mapping of restriction enzyme cleavage sites and a colony harboring a plasmid designated p203-Kan was identified.  p203-Kan had the BamH I fragment 17 ligated to the Tn5-derived kan gene; the T-DNA EcoR I sites being distal to the kan gene and the kan promoter being distal to the T-DNA (Fig. 2).

Example 2  The left end of the disarming deletion

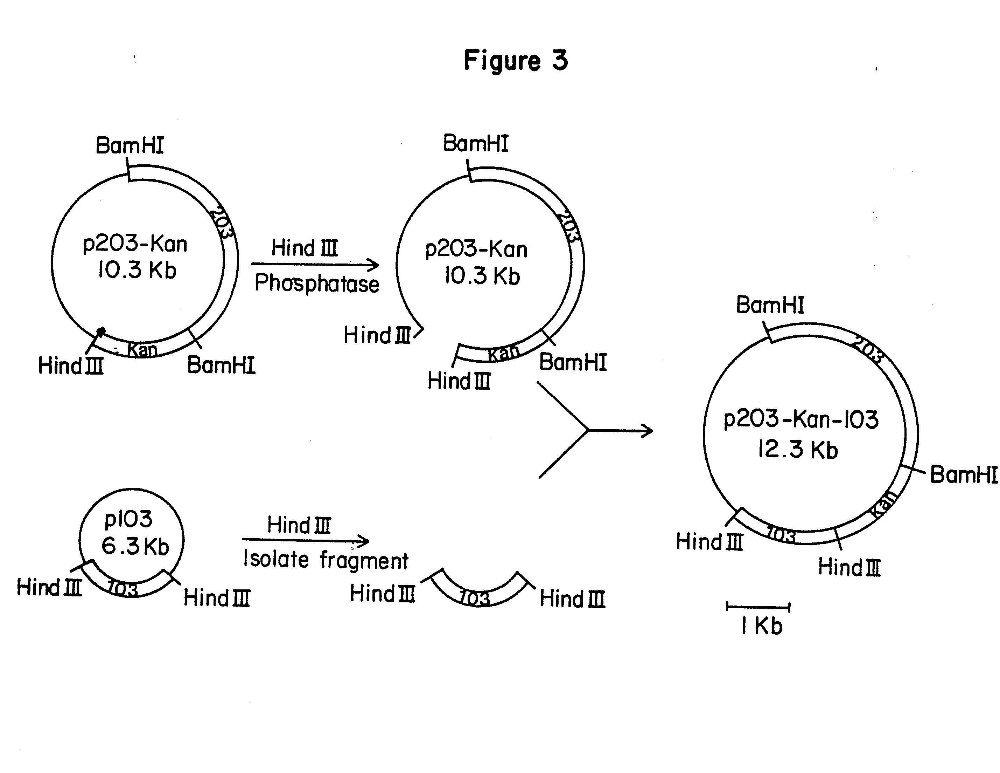

p103 is a plasmid having Hind III fragment of pTi15955 (2.1 kbp) inserted into the Hind III site of pBR322.  p103 was digested with Hind III and the 2.1 kbp T-DNA fragment was isolated by elution from an agarose gel after electrophoretic separation.  After the 2.1 kbp fragment was mixed with and ligated to Hind III-linearized, CIAP-digested p203-Kan, the reaction mixture was transformed into HB101.  DNAs isolated from transformants resistant to kanamycin and ampicillin were characterized by restriction mapping and a colony harboring a plasmid designated p203-Kan-103 was identified.  p203-Kan-103 had the T-DNA of p103 inserted into p203-Kan, the p103 sequences and the p203 sequences being in the same relative orientation as they originally did in pTi15955 (Fig. 3).

## Example 3   Transfer to a shuttle vector

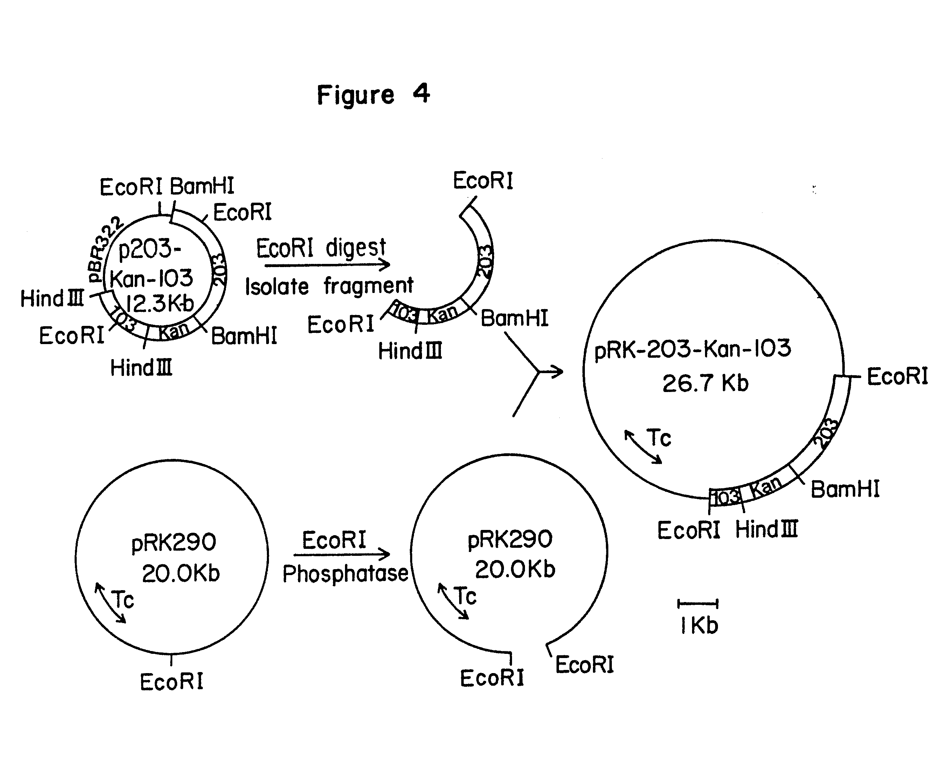

p203-Kan-103 was digested with EcoR I and a 6.6 kbp fragment having the kan gene surrounded by T-DNA was isolated by elution from an agarose gel after electrophoretic separation.  After the 6.6 kbp fragment was mixed with and ligated to EcoR I-linearized, CIAP-digested pRK290, the reaction mixture was transformed into E. coli C600.  DNAs isolated from transformants resistant to tetracycline and kanamycin were characterized by restriction maping and a colony harboring a plasmid designated pRK-203-Kan-103 was identified.  pRK-203-Kan-103 had the EcoR I T-DNA/kan fragment inserted into pRK290 with p103 sequences proximal to the tetracycline resistance gene of pRK290, and carried sequences which constitute a disarming mutation (Fig. 4)

## Example 4   Insertion of plant-expressible genes

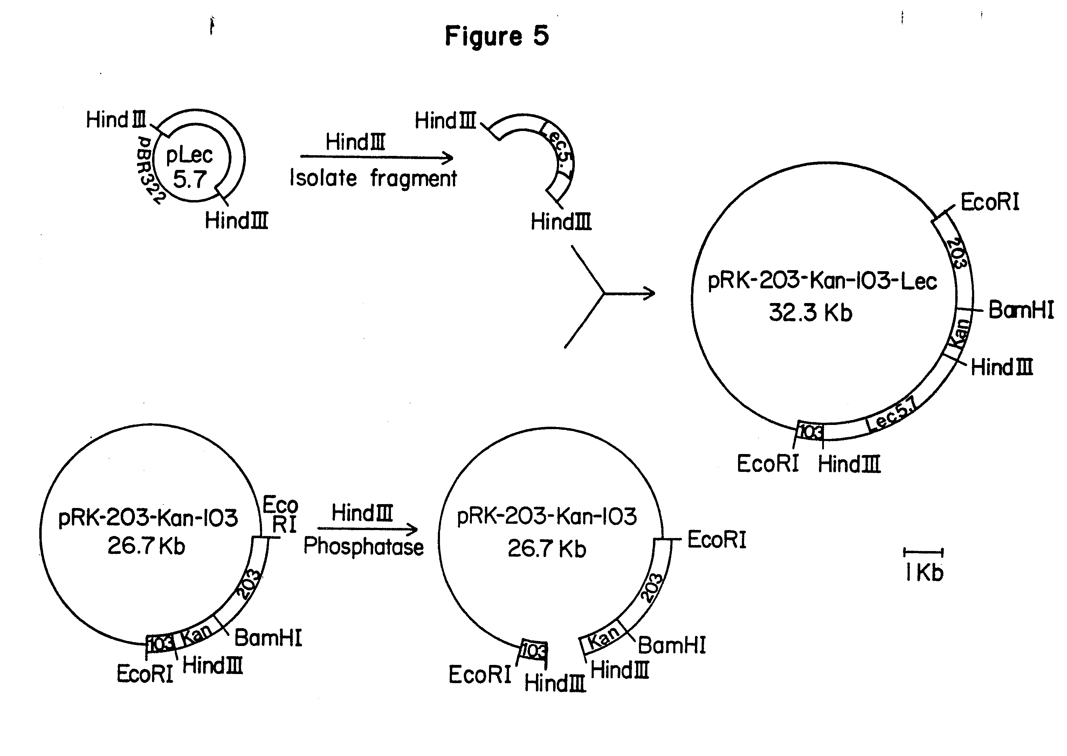

pLec5.7 (L. M Hoffman (1984) J. Mol. Appl. Genet., pλLec5.7 therein) is a plasmid having inserted into the Hind III site of pBR322 a 5.7 kbp Hind III fragment of DNA from the common bean, Phaseolus vulgaris L., encoding a plant-expressible gene for a lectin.  The pLec5.7 coding sequence is identical to that of pPVL134, a lectin cDNA clone.  pLec5.7 was digested with Hind III and a 5.7 kbp fragment of Phaseolus DNA was isolated by elution from an agarsoe gel after electrophoretic separation.  After the 5.7 kbp fragment was mixed with and ligated to Hind III-linearized, CIAP-digested pRK-203-Kan-103, the reaction mixture was transformed into C600.  DNAs isolated from transformants resistant to tetracycline and kanamycin were characterized by restriction mapping and a colony harboring a plasmid designated pRK-203-Kan-103-Lec was identified.  pRK-203-Kan-103-Lec has Phaseolus DNA inserted into pRK-203-Kan-103 between the p103 and the kan sequences, the lectin promoter being proximal to the kan sequences (Fig. 5).

The lectin gene can be removed from pRK-203-Kan-103-Lec by digestion with Hind III, religation to itself, transformation into an appropriate bacterial host, and screening for lack of a 5.7 kbp Hind III fragment, thereby regenerating pRK-203-Kan-103.

The combination of the lectin gene with the disarming mutation exemplifies combination of the mutation with other plant-expressible gene.

Plant-expressible genes on DNA fragments not terminated by Hind III sticky-ends can be inserted in the place of the Phaseolus DNA by other methods which are well understood in the art.  The ends of the DNA to be inserted can be converted to Hind III-ends by use of the appropriate linkers.  Alternatively, the ends of both the insert and the Hind III-linearized pRK-203-Kan-103 can be converted to blunt-ends that can then be joined by blunt-end ligation.  Plant-expressible genes may also be inserted at other locations in pRK-203-Kan-103, e.g. the unique BamH I site at the junction between the pKS4- and p203-derived fragments.  Any restriction site unique to pRK-203-Kan-103 that is found within the pKS4-derived fragment may also be used.  Indeed, any unique sites within the p103- and p203-derived fragments may be used, though the probability of the insert being incorporated by double homologous recombination into a Ti plasmid along with the kan marker decreases as the distance between the insert and the marker is increased, as is well understood in the art.

An alternative to the insertion of a plant-expressible gene into T-DNA at the site of a disarming mutation is insertion elsewhere in the T-DNA.  An example of insertion of a plant-expressible gene into T-DNA has been provided by N. Murai et al. (1983) Science 222:476-482, who have inserted the gene for phaseolin into the structural gene for tml. A. tumefaciens (pTi529R) of Murai et al. is on deposit as NRRL B-15379. The constructions described therein can not readily be combined with those exemplified herein as both use kan as a selectable marker; however, analogous manipulations using combinations of different antibiotic resistance genes to drive marker replacement will be evident to those skilled in the art.  Replacement of kan with a chloramphenicol resistance (cam) gene is exemplified in Example 6, and construction of a deletion lacking a drug resistance marker is exemplified in Example 7.  When tobacco tissue transformed by a disarmed T-DNA/phaseolin gene combination is regenerated into a plant and allowed to flower, high levels of phaseolin sequences are found to be expressed in developing tobacco seeds.

Example 5  Integration of the disarming mutation into pTI15955

pRK-203-Kan-103 and pRK-203-Kan-103-Lec were integrated into pTi15955 using the strategy as described in the Background section on Shuttle Vectors. Triparental matings were generally accomplished as described below; other variations known to those skilled in the art are also acceptable. E. coli C600 (pRK290-based shuttle vector) was mated with E. coli RR1 (pRK2013) and ATCC 15955, an A. tumefaciens strain harboring the octopine-type Ti plasmid pTi15955. The pRK2013 was transferred to the shuttle-vector-carrying strain and mobilized the shuttle vector for transfer to the Agrobacterium. Growth on a minimal medium incapable of supporting the growth of E. coli, AB glucose or AB sucrose, containing both tetracycline and the drug to which the shuttle vector is resistant, often either kanamycin or chloramphenical, resulted in the selection of Agrobacterium cells containing shuttle vector sequences. A mating of these cells with E. coli (pPH1J1), strain 2104, resulted in the transfer of pPH1J1 to the Agrobacterium cells. pPH1J1 and pRK290-based shuttle vectors cannot coexist for long in the same cell. Growth on 100 mg/l gentamycin and either kanamycin or chloramphenical selected for cells which have Ti plasmids that had undergone single-or double-homologous recombination events (cointegration or homogenotization, respectively) with the shuttle vector and now carried the desired construction. The selected colonies were then screened for tetracycline sensitivity, sensitive strains being homogenotes. Commonly used antibiotic concentrations were as follows: 25 mg/l or 10 mg/l tetracycline in E. coli and 5 mg/l tetracycline Agrobacterium, 25 mg/l kanamycin, and 25 mg/l chloramphenicol. E. coli and A. tumefaciens strains were usually grown in L-broth at 37°C and 28°C, respectively. pRK290 and pRK2013 were disclosed by G. Ditta et al. (1980) Proc. natl. Acad. Sci. USA 77:7347-7357, and pPH1J1 by P. R. Hirsh (1978) Thesis, Univ. E. Anglia. Other octopine-type Ti plasmids, e.g. pTiA6, pTiA66, etc., which are homologous to pTi15955, may be substituted for that plasmid; indeed, any TIP T-DNA having enough homology to permit marker exchange may be substituted. The pTi15955 derivatives of pRK-203-Kan-103 and pRK-203-Kan-103-Lec are respectively designated herein as pTi15955-RS-Kan and pTi15955-RS-Kan-Lec.

## Example 6  Replacement of kan with cam

pBR325 (F. Bolivar (1978) Gene 4:121-136) DNA, having been grown in E. coli GM48 (a dam⁻ host that does not methylate DNA in a manner incompatible with the action of Bcl I, M. G. Marinus (1973) Molec. Gen. Genet.

127:47-55), was digested with Bcl I and BamH I, religated to itself, and transformed into E. coli Lucy (M. A. Zarowitz (1983) Ph.D. thesis, Univ. Missouri-Columbia). DNA isolated from transformants resistant to ampicillin and chloramphenicol but sensitive to tetracycline were characterized by restriction analysis, and a colony harboring a plasmid designated pBR325aBB, lacking the Bcl I/BamH I fragment of pBR325, was identified. The Bcl I/BamH I suture of pBR325aBB is not susceptable to the action of either Bcl I or BamH I. After pBR325aBB DNA was digested with Hinc II, the digestion mixture was subjected to agarose gel electrophoresis and a 1.8 kbp Hinc II fragment carrying a chloramphenicol resistance (cam) gene eluted from the gel. After pUC19 DNA (J. Norrander et al. (1983) Gene 26:101-106) digested with Hinc II and CIAP was mixed with and ligated to the Hinc II cam fragment, the ligation mixture was transformed into K802. Plasmid DNAs isolated from chloramphenicol resistant transformants were characterized by gel electrophoresis and a colony harboring a plasmid designated pUCam19, having a cam gene inserted into the pUC19 polylinker, was identified.

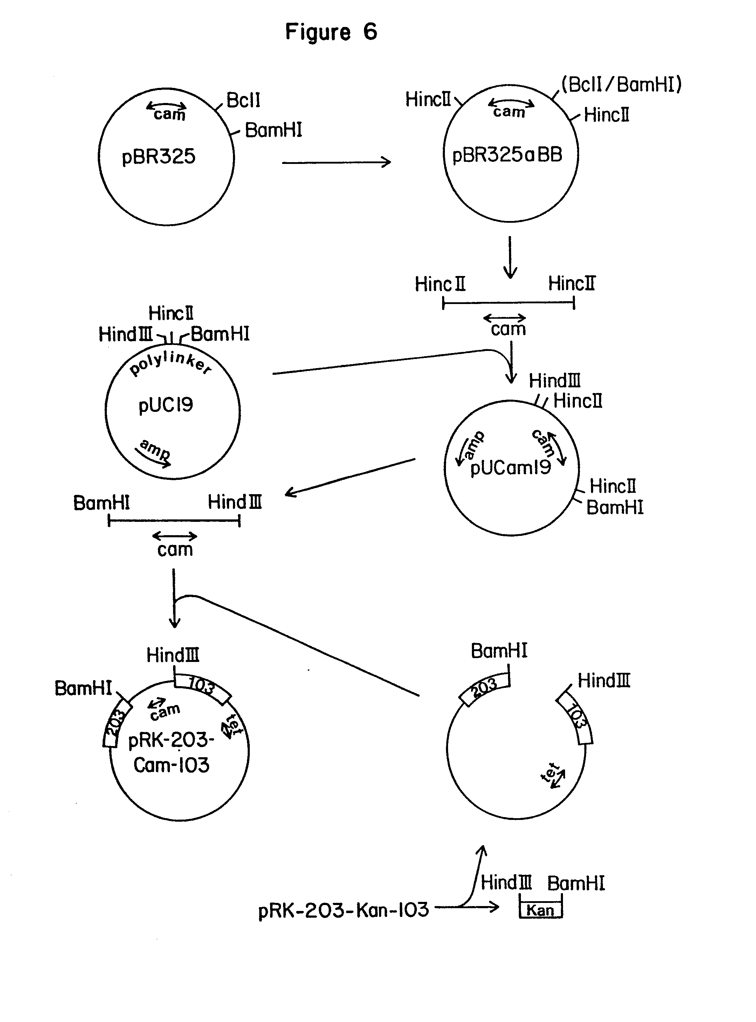

pUCam19 DNA digested by BamH I and Hind III was subjected to agarose gel electrophoresis and a 1.8 kbp fragment carrying a cam gene was eluted from the gel. After the cam fragment was mixed with and ligated to pRK290-203-Kan-103 that had been digested with BamH I, Hind III, and CIAP, the ligation mixture was transformed into E. coli RR1 and selected for chloramphenicol resistant transformants. Plasmid DNAs from such transformants were characterized by restriction mapping and a colony harboring a plasmid having the pBR325 cam gene substituted for the pKS4 kan gene of pRK-203-Kan-103 was identified. The cam-substituted plasmid is herein designated pRK-203-Cam-103 (Fig. 6).

The disarmed mutation was introduced into the kan-disarmed Ti plasmid pTi15955-RS-Kan by the protocol of Example 5. A. tumefaciens cells selected on minimal media containing sucrose, chloramphenicol, and gentamycin were screened for kanamycin sensitivity, thereby detecting replacement by double-homologous recombination of the kan gene with the cam gene. One homogenote was detected among the approximately 500 colonies screened. DNA from a colony sensitive to kanamycin and resistant to chloramphenicol was characterized by restriction mapping and Southern blotting to confirm the incorporation of cam and the elimination of kan. The resultant tmr⁻ tms⁻ Ti plasmid, designated herein as pTi15955-RS-Cam, can be combined with the kan-linked tml-mutants of Murai et al., supra, as discussed in Example 4.


Example 7  Deletion of bacterial drug resistance markers

It is advantageous to have a disarmed mutation without a bacterial drug resistance marker substituted for tmr and tms sequences when one wishes to drive double homologous recombination of several other DNA sequences into a disarmed Ti plasmid, thereby allowing an antibiotic resistance gene otherwise used to disarm the pTi to be used to integrate another non-T-DNA sequence. This Example describes the construction of such a disarming mutation.

pRK-203-Kan-103 or pRK-203-Cam-103 DNA is digested with BamH I and Hind III. After the sticky-ends are converted to blunt-ends by incubation with the Klenow fragment of E. coli DNA polymerase I and all four dNTPs, a method well known in the art, the vector is blunt-end ligated to itself. Alternatively, the linearized vector is mixed with and ligated to a BamH I/Hind III linker, digested with either BamH I or Hind III to remove extra linkers, and then ligated to itself. The reaction mixture resulting from either of the above procedures is transformed into an appropriate host (e.g. C600), selected for resistance to tetracycline, and screened for sensitivity to kanamycin or chloramphenicol, depending on the plasmid starting material. Plasmid DNAs from the selected and screened transformants are characterized by restriction analysis and a colony is identified which harbors a plasmid, designated herein as pRK-203-103, having p203 T-DNA sequences directly joined to p103 T-DNA sequences.

pRK-203-103 is integrated into pTi15955 essentially as described in Example 6 for the integration of pRK-203-Cam-103. pRK-203-103 is transferred as part of a triparental mating into a A. tumefaciens cell harboring either pTi15955-RS-Kan or pTi15955-RS-Cam. After selection of tetracycline resistant cell, introduction of pPH1J1 and selection for gentamycin resistant cells, the transformants are screened for kanamycin or chloramphenicol sensitivity, depending on which disarmed Ti plasmid is used as a starting material. The presence of an antibiotic resistance- less disarming mutation in the pTi is confirmed by restriction analysis and Southern blotting, methods well known in the art. This tmr⁻ tms⁻ Ti plasmid, designated herein as pTi15955-RS, can be combined with the kan-linked tml⁻ mutants of Murai et al., supra, as discussed in Example 4.

## Example 8   Inoculation of Tobacco Stems

Stems of sterile Nicotiana tabacum var. Xanthi were cut into segments approximately 1 cm. long. These segments were placed basal end up in Petri dishes containing Murashige and Skoog medium (MS medium) pH 5.8 without hormonal supplement (Table 1), a medium well known in the art. The basal (upper) ends were then inoculated with A. tumefaciens cells harboring disarmed Ti plasmids by puncturing the cut surface of the stem with a syringe needle. After 3 weeks of incubation at 28°C with 16 hr light and 8 hr dark, callus tissue developed at the upper surface of all stem segments. The callus regions were then transferred to MS medium containing 2.0 mg/l NAA, 0.3 mg/l kinetin and 0.5 mg/ml carbinicillin. After 4 weeks on this medium, the tissues were free of bacteria and could be assayed for the presence of opine, a methodology well understood in the art. Approximately 80% of the tissues were found to be opine positive.

Once free of inciting bacteria, the transformed plant tissues were grown on MS medium at 25°C with 16 hr light and 8 hr dark. These tissues were cloned using a suspension method described by A. N. Binns and F. Meins (1979) Planta 145:365-369. Briefly, tissues were placed in liquid MS medium supplemented with 2.0 mg/l NAA and 0.1 mg/l kinetin, and shaken at 135 rpm at 28°C for 10-14 days. The resultant suspensions were filtered successively through 0.543 and 0.213 mm mesh sieves, concentrated, and plated at a final density of $8 \times 10^3$ cells/ml in MS medium supplemented with 2.0 mg/l NAA and 0.3 mg/l kinetin. After these had grown to approximately 100 mg, colonies were split into 2 pieces. One was placed on complete MS medium and the other was screened for the presence of opines. Approximately 0-50% of the colonies were found to be opine-positive, depending on the particular parental uncloned callus piece from which the colonies were descended. Uncloned pieces having higher concentrations of octopine tended to yield higher percentages of octopine-positive clones.

**0174166**

Example 9  Regeneration of Recombinant Plants

Tissues from various opine-positive clones were transferred onto MS medium supplemented with 0.3 mg/l kinetin and cultured at 28°C with 16 hr light and 8 hr. dark. Shoots initiated were subsequently rooted by placing them in a medium consisting of MS salts without hormones. Rooted plantlets were transferred to soil and placed at high humidity in a greenhouse. After 7-10 days, the plants were then grown with normal greenhouse conditions. All of the regenerated plants derived from opine-positive clones contained opines. The presence of opines indicated thereby that these normal looking plants had been transformed by the disarmed T-DNA.

When screening for expression of lectin sequences, pTi15955-RS-Lec transformed plants are found to have high expression in seeds and low expression in other tissues.

0174166

## Table 1

### MS Medium

| | | |
|---|---|---|
| $NH_4NO_3$ | 1.65 | g/l |
| $KNO_3$ | 1.9 | g/l |
| $CaCl_2 \cdot 2H_2O$ | 440 | mg/l |
| $MgSO_4 \cdot 7H_2O$ | 370 | mg/l |
| $KH_2PO_4$ | 170 | mg/l |
| KI | 0.83 | mg/l |
| $H_3BO_3$ | 6.2 | mg/l |
| $MnSO_4 \cdot 4H_2O$ | 22.3 | mg/l |
| $ZnSO_4 \cdot 7H_2O$ | 8.6 | mg/l |
| $Na_2MoO_4 \cdot 2H_2O$ | 0.25 | mg/l |
| $CuSO_4 \cdot 5H_2O$ | 0.025 | mg/l |
| $CoCl_2 \cdot 6H_2O$ | 0.025 | mg/l |
| $Na_2 \cdot EDTA$ | 37.23 | mg/l |
| $FeSO_4 \cdot 7H_2O$ | 27.85 | mg/l |
| Inositol | 1 | g/l |
| Nicotinic acid | 50 | mg/l |
| Pyroxidine·HCl | 50 | mg/l |
| Thiamine·HCl | 50 | mg/l |
| Sucrose | 30 | g/l |
| Agar | 8 | g/l |

CLAIMS

We claim:

1. A DNA molecule comprising T-DNA sequences, T-DNA sequence positions being numbered by reference to unmodified pTi15955 T-DNA, wherein the T-DNA sequences comprise a disarming mutation, the mutation being a deletion including some but not all tms and tmr sequences and extending

   (a) from a position within about one hundred base pairs of position 5,512 towards tmr and/or

   (b) from a position within about one hundred base pairs of position 9,062 towards tms,

   or a deletion homologous thereto.

2. A DNA molecule according to claim 1, comprising T-DNA sequences, T-DNA sequence positions being numbered and defined by reference to unmodifed pTi15955 T-DNA, wherein the T-DNA sequences comprise a disarming mutation, the mutation being a deletion extending from a position within about one hundred base pairs of position 5,512 to a position within about one hundred base pairs of position 9,062.

3. A DNA according to claim 1, wherein the T-DNA sequences deleted are replaced by a kan or a cam drug resistance marker, thereby substituting a drug resistance marker for the deleted T-DNA.

4. A DNA according to claim 1, additionally comprising a mutation inactivating tml.

5. A DNA according to claim 1, additionally comprising a plant-expressible foreign gene.

6. A DNA according to claim 1, additionally comprising a plant-detectable marker.

7. A DNA according to claim 1, additionally comprising sequences derived from an octopine-type Ti plasmid, pRK290, pBR322, pBR325, or Tn5.

8. A DNA according to claim 7, wherein the octopine-type Ti plasmid is pTi15955.

9. A DNA according to claim 8, wherein the DNA is or is derived from p203-Kan-103, pRK-203-Kan-103, pRK-203-Kan-103-Lec, pRK-203-103, or pRK-203-Cam-103.

10. A bacterium containing the DNA of claim 1.

11. A plant cell transformed to contain the DNA of claim 1.

12. A method for producing transformed plants comprising the steps of:
    (a) transforming a plant cell with T-DNA comprising a disarming mutation; followed by
    (b) regenerating the transformed plant cell or a derivative thereof to form a plant or plant tissue;
    whereby a transformed plant is produced.

13. A method according to claim 12, T-DNA sequence positions being numbered by reference to unmodified pTi15955 T-DNA, wherein the T-DNA sequences comprise a disarming mutation, the mutation being a deletion including some but not all tms and tmr sequences and extending
    (c) from a position within about one hundred base pairs of position 5,512 towards tmr and/or
    (d) from a position within about one hundred base pairs of position 9,062 towards tms,
    or a deletion homologous thereto.

14. A method according to claim 13, T-DNA sequence positions being numbered and defined by reference to unmodified pTi15955 T-DNA, wherein the T-DNA sequences comprise a disarming mutation, the mutation being a deletion extending from a position within about one hundred base pairs of position 5,512 to a position within about one hundred base pairs of position 9,062.

15. A method according to claim 13, wherein the T-DNA is derived from an octopine-type Ti plasmid.

16. A method according to claim 15, wherein the DNA is or is derived from p203-Kan-103, pRK-203-Kan-103, pRK-203-Kan-103-Lec, pRK-203-103, or pRK-203-Cam-103.

17. A method according to claim 12, wherein the T-DNA further comprises a mutation inactivating tml.

18. A method according to claim 12, wherein the T-DNA further comprises a plant-expressible foreign gene.

19. A method according to claim 12, wherein the T-DNA further comprises a plant-detectable marker.

20. A method according to claim 19, further comprising after step (a) the step of:

    (e) screening the transformed cell or derivative thereof for synthesis of an opine.

21. A method according to claim 19, wherein the T-DNA is derived from pTi15955.

22. A method according to claim 12, wherein the plant cell is from a plant of the species _Nicotiana tabacum_.

23. A method according to claim 12, wherein the transformation of step (a) comprises inoculation of inverted stem segments.

24. A method according to claim 12, further comprising after step (a), the step(s) of:

    (f) culturing the transformed cell or its derivative on MS medium or a derivative thereof; and/or

    (g) freeing the transformed cell or derivative thereof from _Agrobacterium_ cells; and/or

    (h) cloning the transformed cell or derivative thereof; and/or (i) culturing the transformed cell or derivative thereof in the presence of an auxin and a cytokinin; and/or

    (j) induction of shoot production by removal of an auxin; and/or

    (k) induction of root production by removal of a cytokinin.

25. A plant cell transformed by the method of claim 12, or a descendant thereof.

## Figure 1

Kilo Base Pairs

Figure 2

# Figure 3

Figure 4

# Figure 5

## Figure 6

European Patent Office

**PARTIAL EUROPEAN SEARCH REPORT**
which under Rule 45 of the European Patent Convention
shall be considered, for the purposes of subsequent
proceedings, as the European search report

**0174166**
Application number

## DOCUMENTS CONSIDERED TO BE RELEVANT

EP 85306159.6

| Category | Citation of document with indication, where appropriate, of relevant passages | Relevant to claim | CLASSIFICATION OF THE APPLICATION (Int. Cl.4) |
|---|---|---|---|
| Y | <u>WO - A1 - 84/02 920</u> (MONSANTO)<br><br>* Claims 7,13; page 30, line 30 - page 31, line 4 * | 1,2 | C 12 N 15/00<br>C 07 H 21/04<br>C 12 P 19/34 |
| A | * Claims 5,9,10,11,14,15–18, 20,21 * | 5,6,10, 11 | C 12 N 1/20<br>C 12 N 5/00 |
| | –– | | //C 12 R 1:41 |
| D,Y | PLANT MOLECULAR BIOLOGY, vol. 2, no. 1, 1983, The Hague, Netherlands<br><br>R.F. BARKER et al. "Nucleobide sequence of the T-DNA region from the Agrobacterium tumefaciens octopine Ti plasmid pTi 15 955" pages 335–350<br><br>* Fig. 1 * | 1,2 | |
| A | –– | 8 | |

**TECHNICAL FIELDS SEARCHED (Int. Cl.4)**

C 12 N
C 07 H
C 12 P

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1–11, 25
Claims searched incompletely: –
Claims not searched: 12–24
Reason for the limitation of the search:

Plant variety, essentially biological process for the production of plants (Art. 53(b) EPC)

| Place of search | Date of completion of the search | Examiner |
|---|---|---|
| VIENNA | 04-12-1985 | FARNIOK |

EPO Form 1505.1 03.82

| Category | Citation of document with indication, where appropriate, of relevant passages | Relevant to claim | CLASSIFICATION OF THE APPLICATION (Int. Cl.4) |
|---|---|---|---|
| | **DOCUMENTS CONSIDERED TO BE RELEVANT** | | |
| D,A | PROCEEDINGS OF THE NATIONAL ACADEMY OF SCIENCES OF THE UNITED STATES OF AMERICA, vol. 80, no. 6, March 1983, Baltimore, USA<br><br>L.W. REAM et al. "Multiple mutations in the T region of the Agrobacterium tumefaciens tumor-inducing plasmid"<br>pages 1660-1664<br><br>  * Totality *<br><br>-- | 1-7,9-11 | |
| D,A | JOURNAL OF MOLECULAR AND APPLIED GENETICS, vol. 1, no. 1, 1981, New York<br><br>A.J.M. MATZKE et al. "Site-specific Insertion of Genes into T-DNA of the Agrobacterium Tumor-Inducing Plasmid: An Approach to Genetic Engineering of Higher Plant Cells"<br>pages 39-49<br><br>  * Page 39 - page 43, column 1, line 10 *<br><br>---- | 3,5-7, 9,10 | TECHNICAL FIELDS SEARCHED (Int. Cl.4) |